# EUROPEAN PATENT SPECIFICATION

(11) **EP 4 299 761 B1**
(45) Date of publication and mention of the grant of the patent: **27.05.2026**
(21) Application number: 22182415.4
(22) Date of filing: 30.06.2022
(51) Int. Cl.: G01N 33/543, C12Q 1/6837, C12Q 1/6825, C12Q 1/70, G01N 21/77, G01N 27/02

(54) **SURFACE-FUNCTIONALIZED OPTICAL ELEMENT**
OBERFLÄCHENFUNKTIONALISIERTES OPTISCHES ELEMENT
ÉLÉMENT OPTIQUE FONCTIONNALISÉ EN SURFACE

(43) Date of publication of application: 03.01.2024
(73) Proprietor: betaSENSE GmbH, 44799 Bochum (DE)
(72) Inventor: Mann, Marvin, 58540 Meinerzhagen (DE); Langenhoff, Lennart Carl, 44789 Bochum (DE); Güldenhaupt, Jörn, 59192 Bergkamen (DE); Wanka, Robin, 44879 Bochum (DE); Kötting, Carsten, 44892 Bochum (DE); Gerwert, Klaus, 48147 Münster (DE)
(74) Representative: Maiwald GmbH

(56) References cited:
- DEVOUGE S ET AL: "Surface functionalization of germanium ATR devices for use in FTIR-biosensors", JOURNAL OF COLLOID AND INTERFACE SCIENCE, ACADEMIC PRESS,INC, US, vol. 332, no. 2, 15 April 2009 (2009-04-15), pages 408 - 415, XP025994723, ISSN: 0021-9797, [retrieved on 20090227], DOI: 10.1016/J.JCIS.2008.12.045
- KLEIREN EMILIE: "TOWARDS AN EARLY DIAGNOSIS OF ALZHEIMER'S DISEASE : DEVELOPMENT OF AN ATR-FTIR BIOSENSOR FOR THE DETECTION OF A� TOXIC CONFORMATIONS", TH�SE UNIVERSIT� LIBRE DE BRUXELLES,, 1 September 2013 (2013-09-01), pages 1 - 162, XP007923061

## Description

### TECHNICAL FIELD

The present invention relates to the field of surface-functionalized optical elements such as surface-functionalized ATR-IR waveguides and optical biosensors which comprise such surface-functionalized optical elements. The surface-functionalized optical element and the biosensor comprising the same can be used in, but is not limited to, a method for detecting a target analyte.

### BACKGROUND

Surface-functionalized optical elements find widespread use in biosensing applications or biosensors. In said applications, a molecular probe is immobilized on the surface of the optical element. The immobilized molecular probe can specifically bind to a target analyte. The chemical/(biochemical) interaction between the molecular probe and the target analyte produces a measurable signal that is specific for the respective target analyte. The measured signal results from a physical-chemical change, which can be measured by optical spectroscopy via the optical element which serves as a so-called transduction element.

For example, WO 2015/121339 A1 describes an infrared sensor element for the direct analysis of the quantity and secondary structure of a candidate biomarker protein undergoing conformational transitions associated with disease progression. Said infrared sensor element comprises a germanium internal reflection element, and at least one receptor for the biomarker protein being an antibody capable of specific and conformationally independent binding to the candidate biomarker protein and being directly grafted to at least one surface of said internal germanium reflection element by silanization with short silane linkers or by thiolation with short thiol linkers, reacting freely accessible amine groups of said at least one receptor with amine-reactive groups on the short silane/thiol linkers, and blocking remaining amine-reactive groups on the short silane/thiol linkers with a blocking substance not cross-reacting with the candidate biomarker protein

In known surface-functionalized optical elements, the functional groups and/or molecular probes can be insufficiently immobilized on the surface of the optical elements. Insufficient immobilization can lead to a direct or delayed detachment of the functional groups and/molecular probes from the surface optical element, e.g. during washing or measuring steps under continuous flow of a solvent over the functionalized surface. As a consequence, the lifetime and functionality of the functionalized surface can decrease.

There is a continuous need in the art to provide surface-functionalized optical elements, which are stable and/or show decreased detachment of functional groups from the surface.

### SUMMARY OF INVENTION

In a first aspect of the invention, a surface-functionalized optical element is provided, comprising
an IR-transparent optical element,
wherein at least a part of the surface of the IR-transparent optical element is bound to a molecular probe by a linker construct,
wherein the linker construct comprises
a surface attachment moiety,
a spacer moiety which comprises a poly(alkylene oxide), and
a peptide moiety, which is covalently bound between the spacer moiety and the molecular probe.

In a second aspect of the invention, a surface-functionalized optical element is provided, comprising an IR-transparent optical element,
wherein at least a part of the surface of the IR-transparent optical element is bound to a molecular probe by a linker construct,
wherein the linker construct comprises
a surface attachment moiety,
a spacer moiety which comprises a poly(alkylene oxide) and a ligation group, which is covalently bound between the poly(alkylene oxide) of the spacer moiety and the molecular probe,
and wherein the ligation group is an optionally substituted N-heterocyclic group having a structure according to any one of formula (HET-I) to (HET-VI): 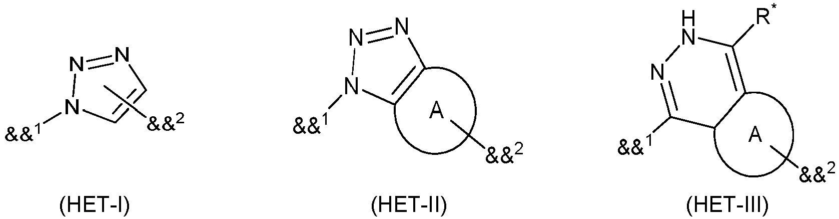 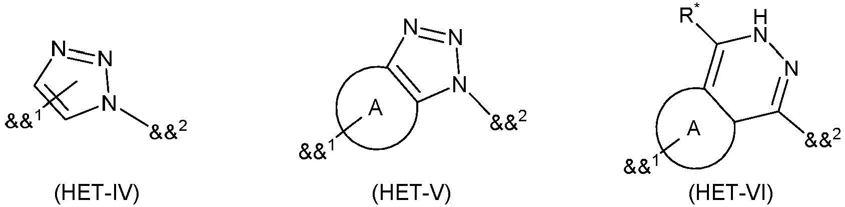
wherein
&&¹ represents attachment to a linking unit of the spacer moiety or to the poly(alkylene oxide),
&&² represents the attachment to a linking unit of the spacer moiety, another moiety of the linker construct or to the molecular probe,
R* is H, an optionally substituted alkyl group or an optionally substituted aryl group, and
ring A is an optionally substituted 8-membered carbocycle or an optionally substituted 8-membered heterocycle.

The inventors surprisingly found that, by binding a molecular probe to the surface of the optical element by the specific linker construct as defined herein, the surface-functionalized optical element has an improved stability. For example, the surface-bound functional groups are less prone to detach from the optical element than in a comparable surface-functionalized optical element which uses a different linker for binding the molecular probe to the surface. Thereby, the functionality and lifetime of the surface-functionalized optical element of the invention is improved.

In another aspect of the invention, an optical biosensor is provided which comprises the surface-functionalized optical element as defined herein.

In another aspect of the invention, a process for preparing the surface-functionalized optical element according to the first aspect of the invention is provided.

In another aspect of the invention, a method for detecting a biomarker is provided. The method comprises the steps of
(i) bringing into contact a surface-functionalized optical element as defined herein with a sample suspected to comprise a target analyte, and optionally wherein the sample is a human body fluid;
(ii) detecting the biomarker based on an interaction between the molecular probe and the target analyte, and optionally wherein the interaction between the molecular probe and the target analyte is detected by infrared spectroscopy, and optionally wherein the biomarker is based on an amide I band position of the target analyte.

In yet another aspect of the invention, the use of a surface-functionalized optical element as defined herein or an optical biosensor as defined herein is provided for one or more of:
a companion diagnostic test,
diagnosing a protein misfolding disease, diabetes or a tumor in a patient,
monitoring of therapy of patients having a protein misfolding disease, diabetes or a tumor, and
for screening of drugs for the treatment of a protein misfolding disease, diabetes or a tumor.

Where the term "comprising" is used in the present description and claims, it does not exclude other non-specified elements of major or minor functional importance. For the purposes of the present invention, the term "essentially consisting of" and "consisting of" are considered to be specific embodiments of the term "comprising of". If hereinafter a group is defined to comprise at least a certain number of features or embodiments, this is also to be understood to disclose a group, which optionally essentially consists only of these features or embodiments or consists only of these features or embodiments.

Whenever the terms "including" or "having" are used, these terms are meant to be equivalent to "comprising" as defined above. Where an indefinite or definite article is used when referring to a singular noun, e.g. "a", "an" or "the", this includes a plural of that noun unless something else is specifically stated. The term "obtained" does not mean to indicate that, e.g., an embodiment must be obtained by, e.g., the sequence of steps following the term "obtained" even though such a limited understanding is always included by the terms "obtained" as a preferred embodiment.

### DETAILED DESCRIPTION

### Surface-functionalized optical element

In a first aspect of the invention, a surface-functionalized optical element is provided. The surface-functionalized optical element comprises
an IR-transparent optical element,
wherein at least a part of the surface of the IR-transparent optical element is bound to a molecular probe by a linker construct,
wherein the linker construct comprises
a surface attachment moiety,
a spacer moiety which comprises a poly(alkylene oxide), and
a peptide moiety, which is covalently bound between the spacer moiety and the molecular probe.

In a second aspect of the invention, a surface-functionalized optical element is provided, comprising an IR-transparent optical element,
wherein at least a part of the surface of the IR-transparent optical element is bound to a molecular probe by a linker construct,
wherein the linker construct comprises
a surface attachment moiety,
a spacer moiety which comprises a poly(alkylene oxide) and a ligation group, which is covalently bound between the poly(alkylene oxide) of the spacer moiety and the molecular probe,
and wherein the ligation group is an optionally substituted N-heterocyclic group having a structure according to any one of formula (HET-I) to (HET-VI) as defined in appended claim 5.

The surface-functionalized optical element can comprise additional elements such as a matrix silane as defined herein or one or more incomplete linker constructs. "Incomplete linker constructs" can be understood as side products which are bound to the surface during preparation of the surface-functionalized optical element, but which do not contain all moieties of the linker construct (e.g. because their reactive functionality has been quenched during preparation) or which are not bound to a molecular probe.

According to one embodiment, the surface-functionalized optical element is an IR-transparent optical element, wherein at least a part of the surface of the IR-transparent optical element is bound to a molecular probe by a linker construct as defined herein, and optionally wherein at least a part of the surface of the IR-transparent optical element is covalently bound to a matrix silane as defined herein. According to one embodiment, the surface-functionalized optical element is an IR-transparent optical element, wherein at least a part of the surface of the IR-transparent optical element is bound to a molecular probe by a linker construct as defined herein, and wherein at least a part of the surface of the IR-transparent optical element is covalently bound to a matrix silane as defined herein, and optionally wherein at least a part of the surface of the IR-transparent optical element is bound to incomplete linker constructs.

### IR-transparent optical element

The surface-functionalized optical element comprises an IR-transparent optical element.

The IR-transparent optical element may be any optical element which is suitable for binding an organic linker compound to its surface.

The IR-transparent optical element may be an optical element which is suitable for use in an optical biosensor. According to one embodiment, the IR-transparent optical element is an IR-transparent optical element which is suitable for attenuated total reflection (ATR) spectroscopy, transmission spectroscopy, or surface-enhanced infrared absorption (SEIRA) spectroscopy. According to one preferred embodiment, the IR-transparent optical element is suitable for attenuated total reflection infrared (ATR-IR) spectroscopy or transmission infrared spectroscopy.

The IR-transparent optical element is preferably at least transparent to infrared radiation with wavenumbers in a range of 40 to 13000 cm⁻¹, more preferably in the range of 500 to 4000 cm⁻¹, even more preferably in the range of 900 to 4000 cm⁻¹, like in the range of 1400 to 1800 cm⁻¹ or 1500 to 1750 cm⁻¹.

According to one preferred embodiment, the IR-transparent optical element is an IR-transparent optical waveguide, like an ATR-IR waveguide. According to one preferred embodiment, the IR-transparent optical element is an ATR-IR waveguide.

According to one embodiment, the IR-transparent optical element is a material and/or crystal selected from the group consisting of plastic, glass, diamond, germanium, silicon, silicon dioxide, silver halides, GaAs, ZnSe, ZnS, thallium(I) mixed halides (e.g. KRS-5), and AMTIRs. "AMTIR" is an amorphous material transmitting infrared radiation which is known in the art. A suitable AMTIR may be Ge₃₃As₁₂Se₅₅ (AMTIR-1).

According to one preferred embodiment, the IR-transparent optical element is an ATR-IR waveguide, which is a silicon crystal or a germanium crystal, and more preferably which is a silicon crystal.

The IR-transparent optical element may be at least partially surface-modified, e.g. by an oxide layer (e.g. silicon dioxide layer). The surface modification can further aid the attachment of the linker construct, e.g. the attachment of a linker construct comprising a surface attachment moiety which is covalently bound to the optical element by a silane group.

Hence, in one embodiment of the invention, at least a part of the IR-transparent optical element has an oxide surface layer, and at least a part of the oxide surface layer is covalently bound to the linker construct. The oxide surface layer is preferably a metal oxide surface layer or a metalloid oxide surface layer. A "metalloid oxide" is an oxide of an element selected from the group consisting of boron, silicon, germanium, arsenic, antimony and tellurium.

More preferably, the metal oxide surface layer or a metalloid oxide surface layer is a silicon dioxide surface layer.

According to one embodiment, at least a part of the IR-transparent optical element has an oxide surface layer, preferably a silicon dioxide surface layer, wherein at least a part of the oxide surface layer is covalently bound to a silane group of the surface attachment moiety of the linker construct.

According to one preferred embodiment, the IR-transparent optical element is an attenuated total reflection infrared (ATR-IR) waveguide, and at least a part of the IR-transparent optical element has an oxide surface layer, and at least a part of the oxide surface layer is covalently bound to the linker construct.

According to one preferred embodiment, the IR-transparent optical element is a silicon crystal or a germanium crystal, wherein the silicon crystal or the germanium crystal has an oxide surface layer, preferably a silicon dioxide surface layer, and wherein at least a part of the oxide surface layer is covalently bound to the linker construct.

### Linker construct

The surface-functionalized optical element comprises a linker construct. At least a part of the surface of the IR-transparent optical element is bound to a molecular probe by the linker construct. The surface of the IR-transparent optical element can be covalently bound to the molecular probe by the linker construct. Hence, according to one embodiment, the linker construct covalently binds the molecular probe to the surface of the IR-transparent optical element.

"Covalently bound" in the meaning of the present disclosure can be understood in that the linker construct comprises at least one continuous chain of sigma-bonds which connects the surface of the IR-transparent optical element to the molecular probe.

According to one embodiment, the linker construct does not contain a coordination transition metal complex, a protein-ligand complex, and a protein-protein complex. A coordination transition metal complex may be, but is not limited to, a histidine nickel complex. A protein-ligand complex may be, but is not limited to, a biotin-avidin or biotin-streptavidin complex. A protein-protein complex may be, but is not limited to, an antibody-antigen complex.

### Surface attachment moiety

The linker construct comprises a surface attachment moiety. The surface attachment moiety covalently attaches the linker construct to the optical element. The surface attachment moiety is not particularly limited as long as the moiety is suitable for attaching the linker construct to the surface of the optical element.

According to one embodiment, the surface attachment moiety comprises a silane group or a sulfide group, and preferably a silane group.

For example, a silane group can be used for attaching the surface attachment moiety to an IR-transparent optical element (e.g. a silicon optical element) having an oxide surface layer such as a metalloid oxide surface layer (e.g. silicon dioxide).

A sulfide group can be used for attaching the surface attachment group to an IR-transparent optical element which is a germanium optical element and/or germanium crystal.

The silane group can have the formula of:

//-O-Si(R¹)₂-A,

wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
// represents the attachment to the surface of the IR-transparent optical element, and
A represents the attachment to the remaining linker construct.

According to one embodiment, the surface attachment moiety has a structure according to formula (S-I):

//*-*Y¹-L¹-X¹-$$¹ (S-I),

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is an optional linking unit,
X¹ is a group selected from -C(O)NH-, -NHC(O)-, -NHC(O)HN-, and -NHC(O)O-,
$$¹ represents attachment to the spacer moiety.

According to one preferred embodiment, the surface attachment moiety has a structure according to formula (S-I):

//*-*Y¹-L¹-X¹-$$¹ (S-I),

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element, and optionally wherein R¹ is each independently selected from C1-C6-alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is a C1-C12 alkyl group, and preferably a C1-C6 alkyl group (e.g. propyl),
X¹ is a group selected from -C(O)NH- and -NHC(O)-, and preferably is -NHC(O)-,
$$¹ represents attachment to the spacer moiety.

According to another embodiment, the surface attachment moiety has a structure according to formula (S-II):

//-Y³-L⁸-$$¹ (S-II),

wherein
// represents the attachment to the surface of the IR-transparent optical element,
Y³ is -S-
L⁸ is a linking unit, and preferably an optionally substituted alkyl group, like an optionally substituted C1-C12 alkyl group,
$$¹ represents attachment to the spacer moiety.

### Spacer moiety

Furthermore, the linker construct comprises a spacer moiety. The spacer moiety comprises a poly(alkylene oxide).

The inventors surprisingly found that the use of a spacer moiety comprising a poly(alkylene oxide) improves the stability of the surface-functionalized optical element compared to a linker construct which does not contain a poly(alkylene oxide). Furthermore, the inventors found that the the use of a spacer moiety comprising a poly(alkylene oxide) improves the stability of the surface-functionalized optical element compared to a linker construct which does not contain a poly(alkylene oxide).

The alkylene subunit of the poly(alkylene oxide) can optionally be substituted. The poly(alkylene oxide) may be, but is not limited to, poly(ethylene oxide), poly(propylene oxide), poly(butylene oxide), or block copolymers thereof.

Preferably, the poly(alkylene oxide) is poly(ethylene oxide), which is also referred to in the art as polyethylene glycol or PEG. According to one preferred embodiment, the poly(alkylene oxide) is a polyethylene glycol having 2 to 100 glycol repeating units, more preferably 2 to 50 repeating units, even more preferably 2 to 25 repeating units, yet even more preferably 2 to 16 repeating units, like 3 to 8 glycol repeating units or 3 to 6 glycol repeating units.

The spacer moiety preferably further comprises a ligation group. The ligation group binds the molecular probe to a linking unit of the spacer moiety, or to another moiety of the linker construct. Preferably, the ligation group is covalently bound between the poly(alkylene oxide) of the spacer moiety and the molecular probe.

The ligation group can be understood as a structural unit which is obtained when preparing the linker construct, and particularly when (i) reacting precursors of the linker construct with one another, (ii) attaching the molecular probe to the linker construct, or (iii) reacting one precursor of the linker construct with a molecular probe which is pre-labeled with another precursor of the linker construct. Further details how the ligation group(s) may be obtained are described below in connection with the process of the invention.

Depending on the structure of the linker construct and its preparation process, the spacer moiety can comprise one or more ligation groups.

The one or more ligation groups can be, but is not limited to, an ester, a hydrazone, an amide, a hydrazone, a succinimide or a ring-opened product of a succinimide, or a group which is obtainable by a bioorthogonal ligation reaction.

The skilled person knows biorthogonal ligation reactions and the chemical structures of the groups which are obtainable by said reactions. Bioorthogonal ligation reactions include, but are not limited to, Staudinger ligations, [2+3]-cycloadditions between azides and alkynes ("click reaction), between azides and cycloalkynes ("copper-free click reaction"), between nitrones and cycloalkynes, [2+4]-cycloadditions between tetrazine and a trans-alkene ("tetrazine ligation"), oxime/hydrazine formation from aldehydes and ketones, and the like. Hence, the bioorthogonal ligation reaction is preferably one of the following reactions: (i) a reaction between an azide and a phosphine or phosphite; (ii) a reaction between an azide and an alkyne (including cycloalkynes); (iii) a reaction between an azide and a cycloalkyne; (iv) a reaction between a tretrazine and a trans-alkenes; (v) an oxime- and/or hydrazone-forming reaction.

In one embodiment, the spacer moiety comprises a ligation group which is covalently bound to the molecular probe. For example, the ligation group can be an ester, a hydrazone, an amide, a succinimide, or a ring-opened product of a succinimide, which optionally has the one of the following structures: 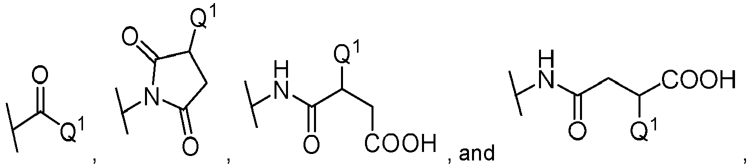 wherein Q¹ wherein Q¹ is a NH, O or S atom(s) of the molecular probe. If the molecular probe contains amino acids (e.g. when the molecular as an antibody), Q¹ may be NH, O or S of an amino acid of the molecular probe. For example, Q¹ may be an NH of a lysine side chain.

In one embodiment, the spacer moiety comprises a ligation group which is obtainable by a bioorthogonal ligation reaction. According to the second aspect of the invention and one embodiment of the first aspect of the invention, the spacer moiety comprises a N-heterocyclic group, which is optionally selected from the group consisting of triazole groups, dihydropyridazine groups, a pyridazine groups, and isoxazoline groups.

According to one preferred embodiment, the spacer moiety comprises a N-heterocyclic group, which is selected from the group consisting of triazole groups, dihydropyridazine groups, a pyridazine groups, and isoxazoline groups. Preferably, the N-heterocyclic group is a triazole group. Preferably, the N-heterocyclic group is covalently bound between the poly(alkylene oxide) of the spacer moiety and the molecular probe.

According to the second aspect of the invention and one embodiment of the first aspect of the invention, the spacer moiety comprises a ligation group which is an optionally substituted N-heterocyclic group having a structure according to any one of formula (HET-I) to (HET-VI): 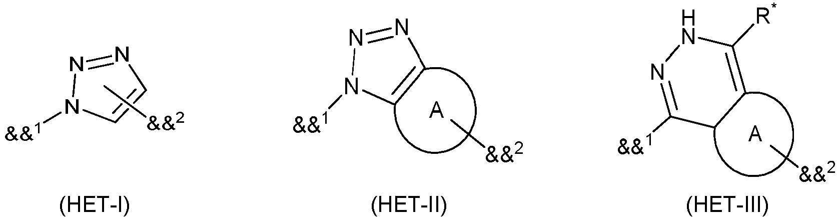 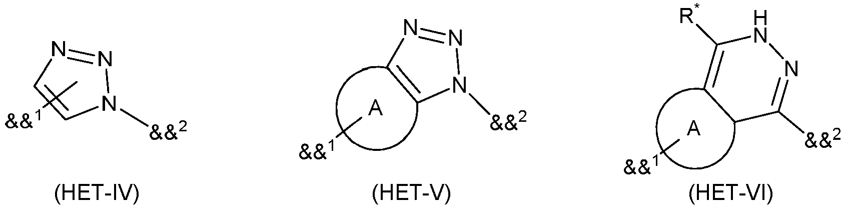 wherein
&&¹ represents attachment to a linking unit of the spacer moiety or to the poly(alkylene oxide),
&&² represents the attachment to a linking unit of the spacer moiety, another moiety of the linker construct or to the molecular probe, and
ring A is an optionally substituted 8-membered carbocycle or an optionally substituted 8-membered heterocycle, and preferably an optionally substituted dibenzo-fused 8-membered N-heterocycle; and
preferably the optionally substituted N-heterocyclic group has the structure according to formula (HET-II) or (HET-V): 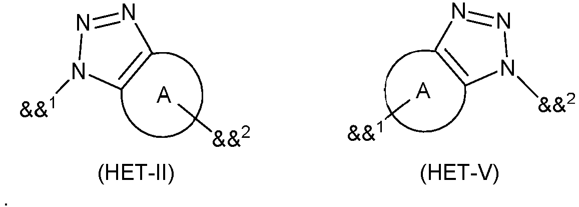
According to one embodiment, the spacer moiety has a structure according to formula (SP-II)

$$²-L²-X²-L³-H¹-L⁴-§§¹ (SP-II),

wherein
$$² represents attachment to the surface attachment moiety,
L² to L⁴ is each independently an optional linking unit,
X² is -(OCH₂CH₂)n¹-, wherein n1 is an integer from 2 to 100, preferably from 2 to 50, more preferably from 2 to 25, and yet even more preferably from 2 to 16 (e.g. 3 to 6),
H¹ is an optionally substituted N-heterocyclic group having a structure according to any one of formula (HET-I) to (HET-VI): 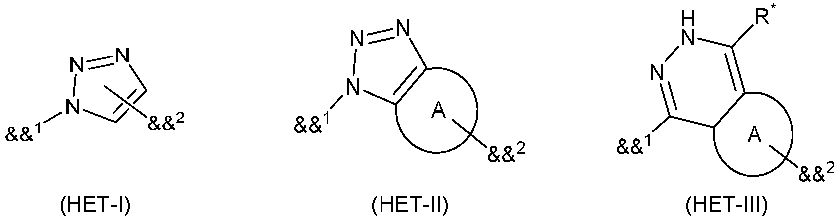 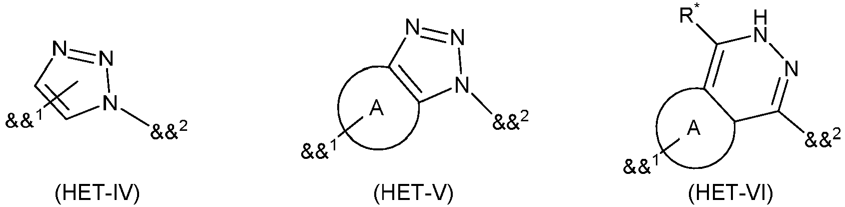 wherein
   &&¹ represents attachment to L³ or, if L³ is absent, corresponds to X²,
   &&² represents the attachment to L⁴ or, if L⁴ is absent, corresponds to §§¹,
   R* is H, an optionally substituted alkyl group or an optionally substituted aryl group, and preferably is a C1-C6 alkyl group (e.g. methyl);
   ring A is an optionally substituted 8-membered carbocycle or an optionally substituted 8-membered heterocycle, and preferably an optionally substituted dibenzo-fused 8-membered N-heterocycle;
   §§¹ represents the attachment to another moiety of the linker construct or to the molecular probe, and
   preferably the optionally substituted N-heterocyclic group has the structure according to formula (HET-II) or (HET-V):

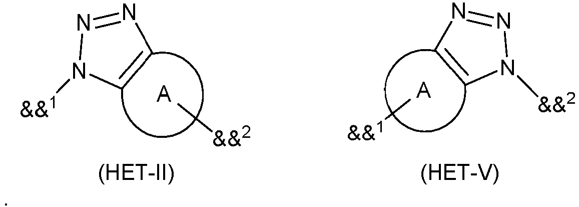

### Peptide moiety

According to the first aspect of the invention and one embodiment of the second aspect of the invention, the linker construct comprises a peptide moiety, which is covalently bound between the spacer moiety and the molecular probe. The peptide moiety functions as a blocking layer or as a part of a blocking layer. It is to be understood that the blocking layer can be formed at least in parts by the peptide moieties of a plurality of linker constructs bound to the surface of the IR-transparent optical element.

It has surprisingly been found by the inventors that covalently binding a peptide moiety between the spacer moiety and the molecular probe increases the inertness of the surface-functionalized optical element towards non-specific binding. The peptide moiety can function as a blocking moiety or, together with other peptide moieties of the surface, as a blocking layer. It has further been found that including a peptide moiety into the linker construct can make conventional blocking of the surface-functionalized optical element unnecessary. The peptide moiety can also function as a synthetic handle or synthetic platform for controlled binding of the molecular probe.

The peptide moiety comprises a peptide compound. A "peptide compound" in the meaning of the present invention is a compound comprising at least two, and optionally at least four, amino acids which are linked by a peptide bond. In this context, "amino acid" is to be understood in a broad way in that it is a compound comprising a carboxylic acid group and an amino group.

The peptide compound is preferably selected from the group consisting of peptides, proteins, protein fragments, each of which is optionally substituted. The optional substituent can be, but is not limited to, a PEG group. Thus, the peptide compound may be a substituted peptide such as a PEGylated peptide.

The peptide compound is preferably a peptide compound which has no or essentially no binding affinity for the target analyte of the molecular probe or a fluid containing said target analyte such as a body fluid (e.g. blood plasma or CSF). The peptide compound is preferably a peptide compound which is inert towards the target analyte of the molecular probe or a fluid containing the target analyte such as a body fluid (e.g. blood plasma or CSF).

The peptide compound may be an optionally substituted natural, semi-synthetic or synthetic peptide, optionally having an amino acid chain length between 2 to 600 amino acids, optionally 2 to 400 amino acids, optionally 2 to 200 amino acids, optionally 2 to 100 amino acids. The peptide compound may be a natural, semi-synthetic or a synthetic peptide having an amine acid chain length between 2 to 100 amino acids (e.g. 5 to 100 amino acids or 10 to 100 amino acids). Preferably, the peptide compound comprises one or more amino acids, which contain an H₂N-, HO- or HS-group in its/their side chain, and more preferably an H₂N-group, e.g. of a lysine chain side.

The peptide compound can be a fragment of a protein. The fragment of the protein can be obtainable or obtained by protein hydrolysis. Thus, the peptide compound may be obtainable or obtained from a protein hydrolysate. For example, the peptide compound can be obtainable or obtained from a hydrolysate of albumin, casein, BSA, serum proteins (e.g. animal serum such as swine, horse, or goat serum), milk proteins, or mixtures thereof. Preferably, the peptide compound is obtainable or obtained from a protein hydrolysate, e.g. a casein hydrolysate.

The peptide compound may be an optionally substituted peptide, optionally having an amino acid chain length between 2 to 600 amino acids, optionally 2 to 400 amino acids, optionally 2 to 200 amino acids (e.g. 2 to 100, 5 to 100, or 10 to 100), wherein the peptide is obtainable or obtained from a protein hydrolysate. The peptide compound may be an optionally substituted peptide having an amino acid chain length between 2 to 600 amino acids, optionally 2 to 400 amino acids, optionally 2 to 200 amino acids (e.g. 2 to 100, 5 to 100, or 10 to 100), wherein the peptide is obtainable or obtained from a protein hydrolysate, wherein the protein is selected from the group of albumin, casein, BSA, serum proteins, milk proteins, and mixtures thereof. The peptide compound may be an optionally substituted peptide having an amino acid chain length between 2 to 100 amino acids (e.g. 5 to 100 or 10 to 100), wherein the peptide is obtainable or obtained from a casein hydrolysate. The casein hydrolysate may be a hydrolysate of high purity casein.

### Structures of the linker construct

The linker construct can have different chemical structures depending on the synthetic procedures for attaching the molecular probe to the surface of the IR-transparent optical element, and depending on the number of moieties of the linker construct.

The linker construct can generally be defined by a structure according to formula (A):

//-(S)-(SP)-\\ (A),

wherein
// represents attachment to the surface of the IR-transparent optical element,
(S) represents the surface attachment moiety,
(SP) represents the spacer moiety, and
\\ represents attachment to another moiety of the linker construct or to the molecular probe.

The spacer moiety (SP) can be directly attached to the molecular probe. Alternatively, the spacer moiety (SP) can be attached to another moiety of the linker construct such as a peptide moiety as defined in connection with the linker structure according to formula (B) below.

According to one embodiment, the surface attachment moiety (S) has a structure according to formula (S-I):

//-Y¹-L¹-X¹-$$¹ (S-I),

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is an optional linking unit,
X¹ is a group selected from -C(O)NH-, -NHC(O)-, -NHC(O)HN-, and -NHC(O)O-,
$$¹ represents attachment to the spacer moiety.

According to one preferred embodiment, the surface attachment moiety (S) has a structure according to formula (S-I):

//-Y¹-L¹-X¹-$$¹ (S-I),

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element, and optionally wherein R¹ is each independently selected from C1-C6-alkoxy, -O-//, and -O-Si^{x}, wherein Si^{x} is a silicon atom of another silicon-containing surface attachment moiety or a silicon atom of an optional matrix silane being attached to the surface of the IR-transparent optical element
L¹ is a C1-C12 alkyl group, and preferably a C1-C6 alkyl group (e.g. propyl),
X¹ is a group selected from -C(O)NH- and -NHC(O)-, and preferably is -NHC(O)-,
$$¹ represents attachment to the spacer moiety.

According to another embodiment, the surface attachment moiety has a structure according to formula (S-II):

//-Y³-L⁸-$$¹ (S-II),

wherein
// represents the attachment to the surface of the IR-transparent optical element,
Y³ is -S-
L⁸ is a linking unit, and preferably an optionally substituted alkyl group, like an optionally substituted C1-C12 alkyl group,
$$¹ represents attachment to the spacer moiety.

It is preferred that the linker construct of formula (A) comprises a surface attachment moiety of formula (S-I).

The spacer moiety (SP) can vary depending on the procedure for (i) synthesizing the linker construct, and/or (ii) attaching the molecular probe to the linker construct. For example, the molecular probe may be reacted with a pre-functionalized surface without pre-labeling of the molecular probe. For example, the molecular probe may be reacted with an aldehyde- , active ester- and/or maleimide-functionalized surface of the optical element.

According to one embodiment, the spacer moiety has a structure according to formula (SP-I)

$$²-L²-X²-L³-Y²-§§¹ (SP-I),

wherein
$$² represents attachment to the surface attachment moiety,
L² and L³ is each independently an optional linking unit,
X² is -(OCH₂CH₂)n¹-, wherein n1 is an integer from 2 to 100, preferably from 2 to 50, more preferably from 2 to 25, and even more preferably 2 to 16 (e.g. 3 to 6),
Y² is selected from the group consisting of an ester, a hydrazone, an amide, a succinimide, and a ring-opened product of a succinimide, and
§§¹ represents the attachment to the molecular probe.

Preferably, Y² of formula (SP-I) has one of the following structures: 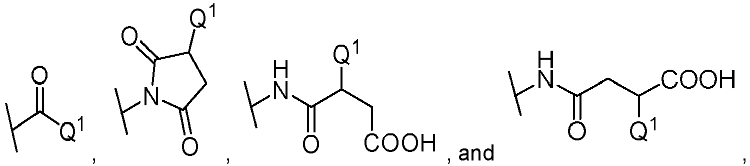 wherein Q¹ wherein Q¹ is a NH, O or S atom(s) of the molecular probe. If the molecular probe contains amino acids (e.g. when the molecular as an antibody), Q¹ may be NH, O or S of an amino acid of the molecular probe. For example, Q¹ may be an NH of a lysine side chain.

The molecular probe may be attached to a pre-functionalized surface by a bioorthogonal ligation reaction. For example, a molecular probe can be ligated to the pre-functionalized surface by a click reaction, which in preferred cases will result in a formation of a N-heterocyclic group in the spacer moiety. To prepare the molecular probe for the bioorthogonal ligation reaction, the molecular probe may be pre-labeled with a group being suitable for a bioorthogonal ligation reaction. However, a pre-labeling of the probe may not be necessary in case the molecular probe itself already contains such a functionality (e.g. an alkyne or azide as part of an unnatural amino acid of a protein).

According to one preferred embodiment, the spacer moiety has a structure according to formula (SP-II)

$$²-L²-X²-L³-H¹-L⁴-§§¹ (SP-II),

wherein
$$² represents attachment to the surface attachment moiety,
L² to L⁴ is each independently an optional linking unit,
X² is -(OCH₂CH₂)n¹-, wherein n1 is an integer from 2 to 100, preferably from 2 to 50, more preferably from 2 to 25, and even more preferably 2 to 16 (e.g. 3 to 6),
H¹ is an optionally substituted N-heterocyclic group having a structure according to any one of formula (HET-I) to (HET-VI): 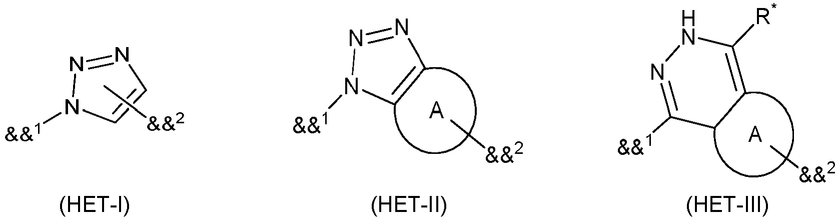 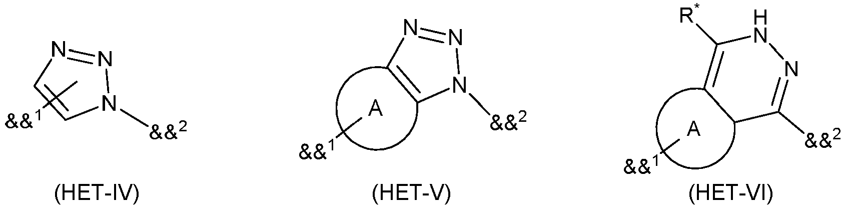
wherein
&&¹ represents attachment to L³ or, if L³ is absent, corresponds to X²,
&&² represents the attachment to L⁴ or, if L⁴ is absent, corresponds to §§¹,
R* is H, an optionally substituted alkyl group or an optionally substituted aryl group, and preferably is a C1-C6 alkyl group (e.g. methyl),
ring A is an optionally substituted 8-membered carbocycle or an optionally substituted 8-membered heterocycle, and preferably an optionally substituted dibenzo-fused 8-membered N-heterocycle;
§§¹ represents the attachment to another moiety of the linker construct or to the molecular probe.

In one embodiment, §§¹ of formula (SP-II) represents the attachment to the molecular probe.

According to one more preferred embodiment, the spacer moiety has a structure according to formula (SP-II)

$$²-L²-X²-L³-H¹-L⁴-§§¹ (SP-II),

wherein
$$² represents attachment to the surface attachment moiety,
L² to L⁴ is each independently an optional linking unit,
X² is -(OCH₂CH₂)n¹-, wherein n1 is an integer from 2 to 100, preferably from 2 to 50, more preferably from 2 to 25, and even more preferably 2 to 16 (e.g. 3 to 6),
H¹ is an optionally substituted N-heterocyclic group having a structure according to formula (HET-II) or (HET-V): 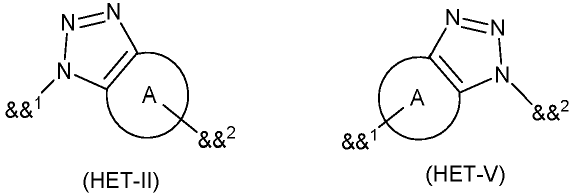
wherein
&&¹ represents attachment to L³ or, if L³ is absent, corresponds to X²,
&&² represents the attachment to L⁴ or, if L⁴ is absent, corresponds to §§¹,
ring A is an optionally substituted 8-membered carbocycle or an optionally substituted 8-membered heterocycle, and preferably an optionally substituted dibenzo-fused 8-membered N-heterocycle;
§§¹ represents the attachment to another moiety of the linker construct or to the molecular probe.

L² can be a short linking unit which connects the surface attachment moiety with the poly(alkylene oxide) of the spacer moiety. L³ can be another short linker which connects the poly(alkylene oxide) with the N-heterocyclic group. For example, L² and L³, if present, can each independently be an alkyl group such as a C1-C16 alkyl group or a C1-C6 alkyl group. Preferably, L² is present and L³ is absent.

L⁴ can be a linking unit which connects the N-heterocyclic group to another moiety of the linker construct, e.g. to the peptide compound of a peptide moiety. Alternatively, L⁴ can be a linking unit which connects the N-heterocyclic group to the molecular probe. For example, L⁴ can be a linking unit which is obtained by reacting an active ester (e.g. NHS-active-ester) or a maleimide with a nucleophilic group of another compound, e.g. a peptide compound, a molecular probe, or a pre-labeled molecular probe, etc. Preferably, L⁴ comprises an ester group, a hydrazone group, an amide group, or a succinimide or a ring-opened product thereof.

According to one embodiment, the spacer moiety has a structure according to formula (SP-Ila) 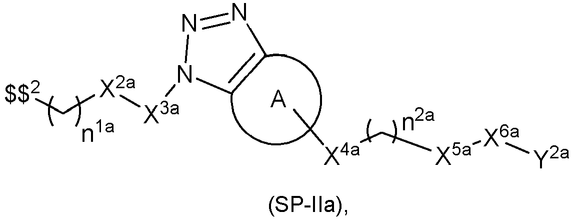 wherein
$$² represents attachment to the surface attachment moiety,
n^{1a} is an integer from 1 to 16, preferably 2 to 4 (e.g. 2);
X^{2a} is -(OCH₂CH₂)n^{4a}-, wherein n^{4a} is an integer from 2 to 16 (e.g. 3 to 6);
X^{3a} is a bond or -CH₂-;
ring A is an optionally substituted 8-membered carbocycle or an optionally substituted 8-membered heterocycle, and preferably an optionally substituted dibenzo-fused 8-membered N-heterocycle;
X^{4a} is a bond, O, -C(O)- or a carbon atom which forms a cyclopropyl group with two adjacent carbons of ring A;
n^{2a} is an integer from 1 to 5;
X^{5a} is a bond or -(OCH₂CH₂)n^{5a}-, wherein n^{5a} is an integer from 1 to 16;
X^{6a} is a bond or -(CH₂)n^{6a}-, wherein n^{6a} is an integer from 1 to 5; and
Y^{2a} is an ester, a hydrozone, an amide, or a succinimide or a ring-opened product thereof, each of which is covalently bound to another moiety of the linker construct or to the molecular probe, and preferably Y^{2a} has one of the following structures: 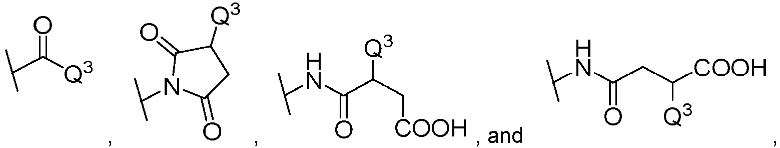
wherein Q³ is a NH, O or S atom(s) of another moiety of the linker construct or of the molecular probe.

According to one embodiment, the spacer moiety has a structure according to formula (SP-IIb) or a [2+3] cycloaddition regioisomer thereof: 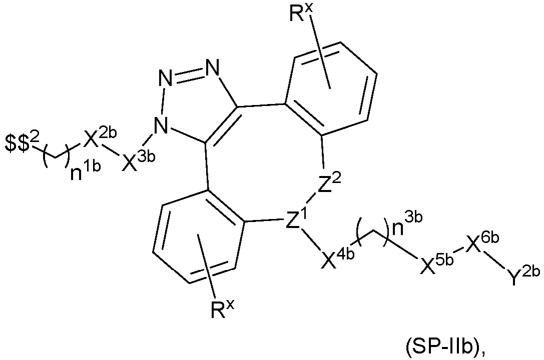 wherein
$$² represents attachment to the surface attachment moiety,
n^{1b} is an integer from 1 to 16, preferably 2 to 4 (e.g. 2);
X^{2b} is -(OCH₂CH₂)n^{4b}-, wherein n^{4b} is an integer from 2 to 16 (e.g. 3 to 6);
X^{3b} is a bond or -CH₂-;
R^{x} is one to four optional substituents (e.g. C1-C6 alkyl groups);
Z¹ is N, O or CH,
Z² is CH₂, CH, or C(O)
X^{4b} is a bond, O, -C(O)- or a carbon atom which forms a cyclopropyl group with Z¹ and Z²;
n^{3b} is an integer from 1 to 5;
X^{5b} is a bond or -(OCH₂CH₂)n^{5b}-, wherein n^{5b} is an integer from 1 to 16;
X^{6b} is a bond or -(CH₂)n^{6b}-, wherein n^{6b} is an integer from 1 to 5; and
Y^{2b} is an ester, an amide, a hydrazone, or a succinimide or a ring-opened product thereof, each of which is covalently bound to another moiety of the linker construct or to the molecular probe, and preferably Y^{2b} has one of the following structures: 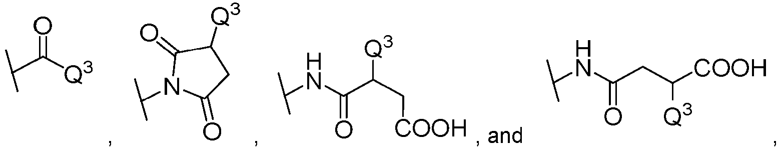
wherein Q³ is a NH, O or S atom(s) of another moiety of the linker construct or of the molecular probe.

According to one embodiment, the spacer moiety has a structure according to formula (SP-Ilc) or a [2+3] cycloaddition regioisomer thereof: 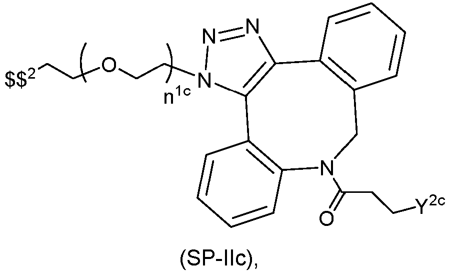 wherein
$$² represents attachment to the surface attachment moiety,
n^{1c} is an integer from 2 to 16 (e.g. 3 to 6); and
Y^{2c} is an ester, an amide, a hydrazone, or a succinimide or a ring-opened product thereof, each of which is covalently bound to another moiety of the linker construct or to the molecular probe, and preferably Y^{2c} has one of the following structures: 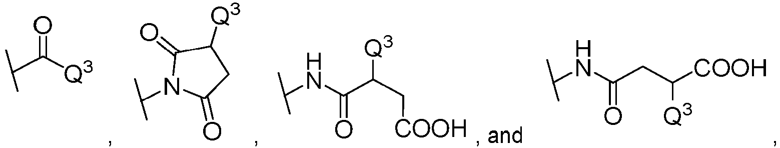
wherein Q³ is a NH, O or S atom(s) of another moiety of the linker construct or of the molecular probe.

In each one of above formula (SP-IIa) to (SP-llc), it is more preferred that Y^{2a}, Y^{2b}, and Y^{2c}, respectively, is 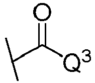 wherein Q³ is a NH or O atom of another moiety of the linker construct or of the molecular probe.

According to the first aspect of the present invention and one embodiment of the second aspect of the present invention, the linker construct further comprises a peptide moiety (Pep) which is covalently bound between the spacer moiety (SP) and the molecular probe. The peptide moiety comprises a peptide compound as defined herein above, and optional linking units which connect the peptide compound to the spacer moiety and to the molecular probe. The linking units may be any linking units which are suitable for attaching a peptide compound to another organic compound. The linking units may be, but are not limited to, e.g. esters, amides, thiolesters, etc.

When a peptide moiety is present in the linker construct, the linker construct can generally be defined by the structure according to formula (B):

//-(S)-(SP)-(Pep)-\\ (B),

wherein
// represents attachment to the surface of the IR-transparent optical element,
(S) represents the surface attachment moiety,
(SP) represents the spacer moiety,
(PEP) is a peptide moiety comprising a peptide compound as defined herein above, and
\\ represents attachment to the molecular probe.

Regarding the definition of the surface attachment group (S) and the spacer moiety (SP) in formula (B), it is referred to the embodiments and preferred embodiments defined herein above in connection with the linker construct according to formula (A).

Preferably, the surface attachment group (S) has a structure according to formula (S-I) and the spacer moiety (SP) has a structure according to any one of formula (SP-II) and (SP-IIa) to (SP-IIc).

It is to be understood that, when a peptide moiety is present in the linker construct, §§¹ of formula (SP-II) represents the attachment to the peptide compound of the peptide moiety.

Furthermore, it is to be understood that, when a peptide moiety is present in the linker construct, each one of Y^{2a} to Y^{2c} of formula (SP-IIa) to (SP-IIc) represents an ester, a hydrazone, an amide, or a succinimide or a ring-opened product thereof, each of which is covalently bound to the peptide compound of the peptide moiety.

According to one embodiment, the peptide moiety (Pep) has a structure according to Formula (PEP-I):

§§²-P¹-L⁵-H²-L⁶-##¹ (PEP-I),

wherein
§§² represents attachment to the spacer moiety (SP),
P¹ is the peptide compound of the peptide moiety,
L⁵ and L⁶ is each independently an optional linking unit,
H² is an optionally substituted N-heterocyclic group having a structure according to any one of formula (HET-VII) to (HET-XII): 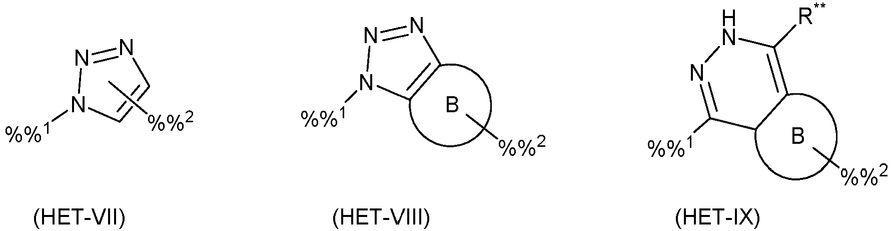 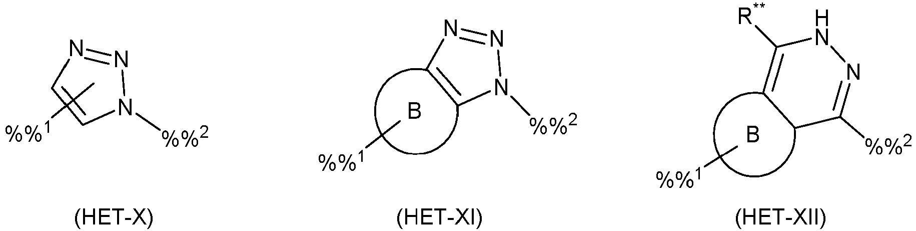
wherein
%%¹ represents the attachment to L⁵ or, when L⁵ is absent, corresponds to Z¹,
%%² represents the attachment to L⁶ or, when L⁶ is absent, corresponds to ##¹,
R** is H, an optionally substituted alkyl group or an optionally substituted aryl group, and preferably is a C1-C6 alkyl group (e.g. methyl),
ring B is an optionally substituted 8-membered carbocycle or an optionally substituted 8-membered heterocycle, and preferably an optionally substituted dibenzo-fused 8-membered N-heterocycle;
##¹ represents attachment to the molecular probe.

According to one embodiment, the peptide moiety (Pep) has a structure according to Formula (PEP-I):

§§²-P¹-L⁵-H²-L⁶-##¹ (PEP-I),

wherein
§§² represents attachment to the spacer moiety (SP),
P¹ is the peptide compound of the peptide moiety,
L⁵ and L⁶ is each independently a linking unit,
H² is an optionally substituted N-heterocyclic group having a structure according to any one of formula (HET-VII) to (HET-XII): 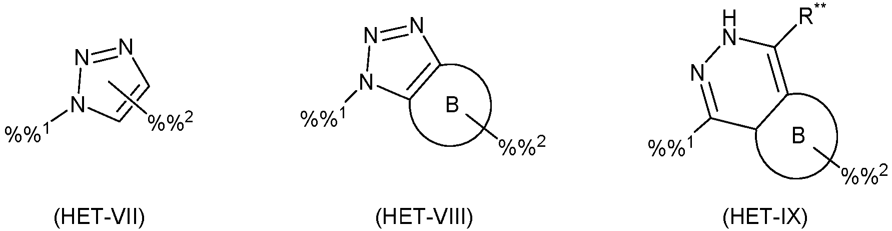 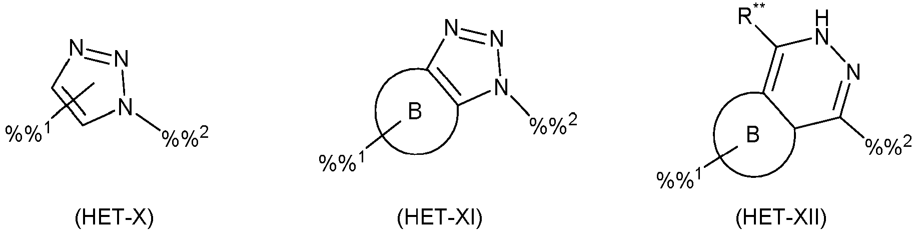
wherein
%%¹ represents the attachment to L⁵,
%%² represents the attachment to L⁶,
R** is H, an optionally substituted alkyl group or an optionally substituted aryl group, and preferably is a C1-C6 alkyl group
ring B is an optionally substituted 8-membered carbocycle or an optionally substituted 8-membered heterocycle, and preferably an optionally substituted dibenzo-fused 8-membered N-heterocycle;
##¹ represents attachment to the molecular probe.

Preferred embodiments of the peptide compound P¹ are described above in connection with the peptide compound of the optional peptide moiety. These embodiments and preferred embodiments are also disclosed in combination with P¹ of formula (PEP-I).

Linking units L⁵ and L⁵ are not particularly limited. Linking units L⁵ and L⁶ can each independently comprise a short alkyl chain (e.g. C1-C6 alkyl chain), and optionally a short PEG group (e.g. a PEG group with 2 to 10 glycol repeating units).

Preferably, L⁵ and L⁶ are each independently linking units which are connected to the peptide compound and/or to the molecular probe by an ester, a hydrazone, an amide, a succinimide or a ring opened product thereof.

According to one embodiment, the peptide moiety (Pep) has a structure according to Formula (PEP-I):

§§²-P¹-L⁵-H²-L⁶-##¹ (PEP-I),

wherein
§§² represents attachment to the spacer moiety (SP),
P¹ is the peptide compound of the peptide moiety, and
"-L⁵-H²-L⁶-##¹" has a structure according to formula (sub-PEP-a): 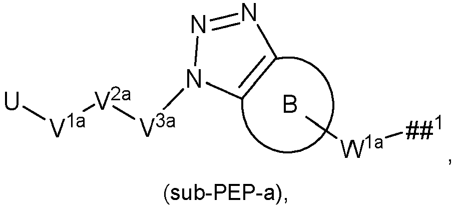
wherein
U is an ester, a hydrazone, an amide, or a succinimide or a ring-opened product thereof, each of which is covalently bound to the peptide compound P¹,
   V^{1a} is (CH₂)m^{1a}, wherein m^{1a} is an integer between 1 to 10, and preferably between 1 and 4,
   V^{2a} is (OCH₂CH₂)m^{2a}, wherein m^{2a} is an integer between 1 to 15, and preferably between 2 to 10, and
   V^{3a} is a bond or CH₂, and preferably a bond,
   W^{1a} is a linking unit,
   ring B is an optionally substituted 8-membered carbocycle or an optionally substituted 8-membered heterocycle, and preferably an optionally substituted dibenzo-fused 8-membered N-heterocycle;
##¹ represents attachment to the molecular probe.

According to one embodiment, the peptide moiety (Pep) has a structure according to Formula (PEP-I):

§§²-P¹-L⁵-H²-L⁶-##¹ (PEP-I),

wherein
§§² represents attachment to the spacer moiety (SP),
P¹ is the peptide compound of the peptide moiety, and
"-L⁵-H²-L⁶-##¹" has a structure according to formula (sub-PEP-b): 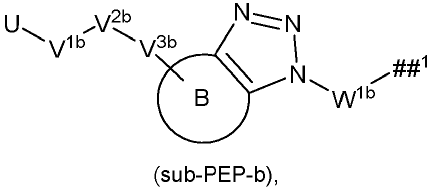
wherein
U is an ester, a hydrazone, an amide, or a succinimide or a ring-opened product thereof, each of which is covalently bound to the peptide compound P¹,
   V^{1b} is (CH₂)m^{1b}, wherein m^{1b} is an integer between 1 to 10,
   V^{2b} is a bond, and
   V^{3b} is C(O),
   W^{1b} is a linking unit,
   ring B is an optionally substituted 8-membered carbocycle or an optionally substituted 8-membered heterocycle, and preferably an optionally substituted dibenzo-fused 8-membered N-heterocycle;
##¹ represents attachment to the molecular probe.

According to one embodiment, the peptide moiety (Pep) has a structure according to Formula (PEP-I):

§§²-P¹-L⁵-H²-L⁶-##¹ (PEP-I),

wherein
§§² represents attachment to the spacer moiety (SP),
P¹ is the peptide compound of the peptide moiety, and
"-L⁵-H²-L⁶-##¹" has a structure according to formula (sub-PEP-c) or a [2+3] cycloaddition regioisomer thereof: 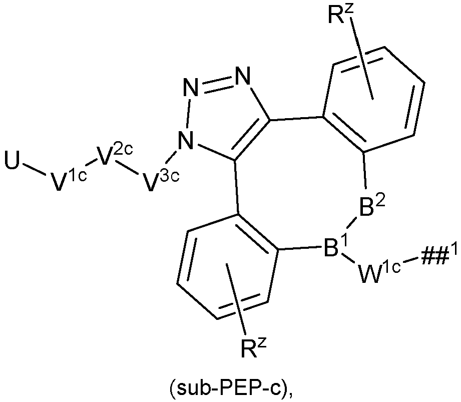
wherein
U is an ester, a hydrazone, an amide, or a succinimide or a ring-opened product thereof, each of which is covalently bound to the peptide compound P¹,
   V^{1c} is (CH₂)m^{1c}, wherein m^{1c} is an integer between 1 to 10, and preferably between 1 and 4,
   V^{2c} is (OCH₂CH₂)m^{2c}, wherein m^{2c} is an integer between 1 to 15, and preferably between 2 to 10, and
   V^{3c} is a bond or CH₂, and preferably a bond,
   W^{1c} is a linking unit,
   R^{z} is from one to four optional substituents, and preferably R^{z} is absent,
   B¹ is N or CH, and preferably N,
   B² is CH₂,
##¹ represents attachment to the molecular probe.

According to one embodiment, the peptide moiety (Pep) has a structure according to Formula (PEP-I):

§§²-P¹-L⁵-H²-L⁶-##¹ (PEP-I),

wherein
§§² represents attachment to the spacer moiety (SP),
P¹ is the peptide compound of the peptide moiety, and
"-L⁵-H²-L⁶-##¹" has a structure according to formula (sub-PEP-d) or a [2+3] cycloaddition regioisomer thereof : 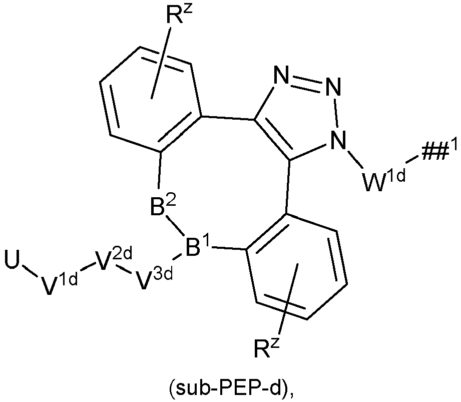
wherein
U is an ester, a hydrazone, an amide, or a succinimide or a ring-opened product thereof, each of which is covalently bound to the peptide compound P¹,
   V^{1d} is (CH₂)m^{1d}, wherein m^{1d} is an integer between 1 to 10,
   V^{2d} is a bond, and
   V^{3d} is C(O),
   W^{1d} is a linking unit, and preferably a linking unit comprising (i) a PEG linker, and (ii) an ester, an amide, or a succinimide or a ring-opened product thereof, each of which is covalently bound to the molecular probe,
   R^{z} is from one to four optional substituents, and preferably R^{z} is absent,
   B¹ is N,
   B² is CH₂,
##¹ represents attachment to the molecular probe.

Preferably, substituent U in any one of the above formula (sub-PEP-a) to (sub-PEP-d) is one of: 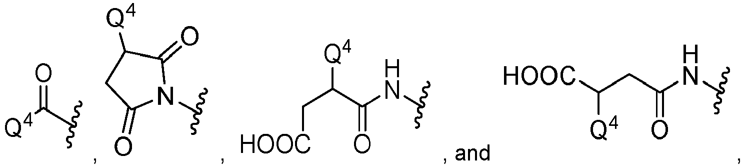 wherein Q⁴ is NH, O or S of an amino acid of the peptide compound P¹.

More preferably, substituent U in any one of the above formula (sub-PEP-a) to (sub-PEP-d) is 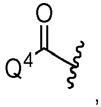 wherein Q⁴ is NH or O of an amino acid of the peptide compound P¹,

### Molecular probe

The surface-functionalized optical element comprises a molecular probe.

The molecular probe can be attached to the linker construct by a ligation group. The ligation group can be, but is not limited to, an ester, a hydrazone, an amide, a hydrazone, a succinimide or a ring-opened product of a succinimide, or a group which is obtainable by a bioorthogonal ligation reaction (e.g. a N-heterocyclic group as defined herein above).

Preferably, the molecular probe is covalently bound to the linker construct by a group which is obtainable by a bioorthogonal ligation reaction, or by an ester, or an amide, or a succinimide or a ring-opened product thereof.

For example, the molecular probe can be covalently bound to the linker construct by one of the following structures: 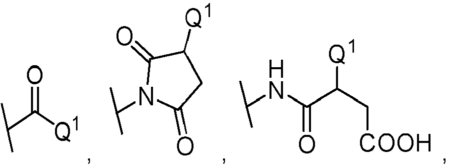 and 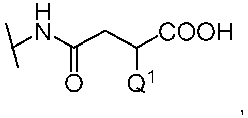 wherein Q¹ is a NH, O or S atom(s) of the molecular probe, and "\" represents the attachment of the remaining linker construct. Preferably, the molecular probe is bound to the linker construct by the following group: : 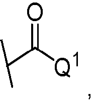 wherein Q¹ is a NH or O atom of the molecular probe, and "\" represents the attachment of the remaining linker construct

The molecular probe is not particularly limited and may be selected by the person of skill according to the target analyte which is to be detected and/or analyzed, and/or the target chemical interaction which is to be detected or analyzed. For example, the molecular probe can be a molecular probe which is known in the art to be useful in ATR-IR-spectroscopy and biosensors using said spectroscopy.

According to one embodiment, the molecular probe is an antigen, an antibody, a fragment of an antibody, an antibody fusion protein or a fusion protein with an antibody fragment, an antibody conjugate or a conjugate with an antibody fragment, a protein complex which optionally contains an antibody fragment or a fusion protein thereof, an anticalin, a nanobody, a small organic molecule, a drug, a nucleic acid, an aptamer, a lipid, a carbohydrate, a peptide, or mixtures thereof.

According to one embodiment, the molecular probe is an antigen-binding protein, optionally selected from an antibody, a fragment of an antibody, an antibody fusion protein or a fusion protein with an antibody fragment, an antibody conjugate or a conjugate with an antibody fragment, or a protein complex which optionally contains an antibody fragment or a fusion protein thereof, an anticalin, a nanobody, and mixtures thereof.

According to one preferred embodiment, the molecular probe is an anti-amyloid β antibody, an anti-alpha synuclein antibody, an anti-tau antibody, an anti-TDP-43 antibody, or a fragment, a fusion protein and/or a conjugate of said antibodies.

The anti-Amyloid β antibody may be selected from the following list: 2E9, H31L21, 11A50-B10, 12B2, 4G8, 11H3, MOAB-2, anti Aβ 25-35, A8978, 5C3, 8G7, 6G12, 1E8, and 32A1. The anti-alpha synuclein antibody may be 4B12, AKS5946,S5566. The anti-tau antibody may be tau-5 or tau-396. The anti-TDP-43 antibody may be 1HCLC.

According to one embodiment, the molecular probe is an anti-amyloid β antibody, or a fragment, a fusion protein, and/or an conjugate thereof.

According to one embodiment, the molecular probe is an anti-alpha synuclein antibody, or a fragment, a fusion protein, and/or an conjugate thereof.

According to one embodiment, the molecular probe is capable of forming a complex with a protein which is associated with or may cause a protein misfolding disease. Protein misfolding diseases, also referred to in the art as proteopathies, are a class of disease in which one or more proteins are misfolded and/or become structurally abnormal, which can disrupt the function of cells, tissues, and/or organs. Protein misfolding diseases and proteins associated with protein misfolding diseases are known to the skilled person and include, but are not limited to, amyloid beta-peptide, tau proteins, alpha synuclein, TDP-43, and Huntingtin.

According to one preferred embodiment, the molecular probe is capable of forming a complex with a target analyte selected from the group consisting of amyloid-beta (Aβ) peptides and isoforms thereof, alpha synuclein, tau protein, TDP-43, human islet amyloid polypeptide (hiAPP), prion protein, and or p53.

The amyloid-beta (Aβ) peptides may be monomeric, oligomeric or fibrillar amyloid-beta (Aβ) peptides.

### Matrix silane

The surface-functionalized optical element can further comprise a matrix silane, wherein at least a part of the surface of the IR-transparent optical element is covalently bound to the matrix silane.

According to one embodiment, the matrix silane comprises a poly(alkylene oxide) group, preferably a poly(ethylene glycol) group, and an inert terminal group. An inert terminal group can be, but is not limited to, a terminal alkyl group such as a terminal C1-C6 alkyl group (e.g. methyl), or a terminal alkoxy group such as a terminal C1-C6 alkoxy group (e.g. methoxy).

The matrix silane can function as a matrix on the surface of the IR-transparent optical element which reduces non-specific binding of media or sample components to the surface, and which further contributes to the stability of the surface-functionalization. Furthermore, the use of a matrix silane allows for fine tuning the amount of linker construct to be bound to the surface of the optical element, and allows for spacing the linker construct on the surface of the optical element. The spacing of the linker construct in turn allows for spacing the molecular probe on the surface of the optical element, which can improve interaction and/or detection between the molecular probe and the target analyte, e.g. between an antibody as molecular probe and a target antigen.

According to one preferred embodiment, at least a part of the surface of the optical element is covalently bound to a matrix silane, wherein the matrix silane has a structure according to formula (MA-a):

//-(S)-(PAO spacer)-(cap) (MA-a)

wherein
"//" represents attachment to the surface of the IR-transparent optical element,
(S) represents a surface attachment moiety,
(PAO spacer) represents a spacer moiety which comprises a poly(alkylene oxide) group, and
(cap) represents an inert terminal group, and preferably a terminal C1-C6 alkyl group (e.g. methyl) or a terminal C1-C6 alkoxy group (e.g. methoxy).

The matrix silane can have the same surface attachment moiety as the linker construct or a different one. According to one embodiment, the matrix silane has the same surface attachment group as the linker construct.

According to one embodiment, the surface attachment moiety (S) of formula (MA-a) has a structure according to formula (S-MA):

//-Y¹¹-L¹¹-X¹¹-$$^{x} (S-MA),

wherein
// represents the attachment to the surface of the IR-transparent optical element,
Y¹¹ is -O-Si(R¹¹)₂-, wherein R¹¹ is each independently selected from H, OH, optionally substituted alkyl, optionally substituted alkoxy, -O-//, and -O-Si^{y}, wherein Si^{y} is a silicon atom of another silicon-containing surface attachment moiety or a silicon atom of an optional matrix silane being attached to the surface of the IR-transparent optical element,
L¹¹ is an optional linking unit,
X¹¹ is a group selected from -C(O)NH-, -NHC(O)-, -NHC(O)HN-, and -NHC(O)O-,
$$^{x} represents attachment to the spacer moiety which comprises a poly(alkylene oxide) group.

According to one preferred embodiment, the surface attachment moiety (S) of formula (MA-a) has a structure according to formula (S-MA):

//-Y¹¹-L¹¹-X¹¹-$$^{x} (S-MA),

wherein
// represents the attachment to the surface of the IR-transparent optical element,
Y¹¹ is -O-Si(R¹¹)₂-, wherein R¹¹ is each independently selected from H, OH, optionally substituted alkyl, optionally substituted alkoxy, -O-//, and -O-Si^{y}, wherein Si^{y} is a silicon atom of another silicon-containing surface attachment moiety or a silicon atom of an optional matrix silane being attached to the surface of the IR-transparent optical element, and optionally wherein R¹¹ is each independently selected from C1-C6-alkoxy, -O-//, and -O-Si^{y}, wherein Si^{y} is a silicon atom of another silicon-containing surface attachment moiety or a silicon atom of an optional matrix silane being attached to the surface of the IR-transparent optical element,
L¹¹ is a C1-C12 alkyl group, and preferably a C1-C6 alkyl group,
X¹¹ is a group selected from -C(O)NH- and -NHC(O)-, and preferably is -NHC(O)-,
$$^{x} represents attachment to the spacer moiety which comprises a poly(alkylene oxide) group.

The spacer (POA spacer) as defined in formula (MA-a) comprises a poly(alkylene oxide) group. The alkylene subunit of the poly(alkylene oxide) can optionally be substituted. The poly(alkylene oxide) may be, but is not limited to, poly(ethylene oxide), poly(propylene oxide), poly(butylene oxide), or block copolymers thereof.

Preferably, the poly(alkylene oxide) is poly(ethylene oxide), which is also referred to in the art as polyethylene glycol or PEG. According to one preferred embodiment, the poly(alkylene oxide) is a polyethylene glycol having 2 to 100 glycol repeating units, more preferably 2 to 50 repeating units, even more preferably 2 to 25 repeating units, yet even more preferably 2 to 16 repeating units, like 2 to 8 glycol repeating units or like 3 to 6 glycol repeating units.

According to one embodiment, at least a part of the surface of the IR-transparent optical element is covalently bound to a matrix silane, wherein the matrix silane has a structure according to formula (MA-b):

//-y¹¹-L¹¹-X¹¹-X¹²-X¹³-T¹ (MA-b),

wherein
// represents the attachment to the surface of the IR-transparent optical element,
Y¹¹ is -O-Si(R¹¹)₂-, wherein R¹¹ is each independently selected from H, OH, optionally substituted alkyl, optionally substituted alkoxy, -O-//, and -O-Si^{y}, wherein Si^{y} is a silicon atom of another silicon-containing surface attachment moiety or a silicon atom of an optional matrix silane being attached to the surface of the IR-transparent optical element, and optionally wherein R¹¹ is each independently selected from C1-C6-alkoxy, -O-//, and -O-Si^{y}, wherein Si^{y} is a silicon atom of another silicon-containing surface attachment moiety or a silicon atom of an optional matrix silane being attached to the surface of the IR-transparent optical element
L¹¹ is a C1-C12 alkyl group, and preferably a C1-C6 alkyl group,
X¹¹ is a group selected from -C(O)NH- and -NHC(O)-, and preferably is -NHC(O)-,
X¹² is a bond or an alkyl group, and preferably is a C1-C6 alkyl group,
X¹³ is-(OCH₂CH₂)k-, wherein k is an integer from 2 to 16 (e.g. 3 to 6);
T¹ is an alkyl group or an alkyloxy group, and preferably a C1-C6 alkyl group (e.g. methyl) or a C1-C6-alkoxy group (e.g. methoxy).

### Optical Biosensor

In one aspect, the invention provides an optical biosensor which comprises the surface-functionalized optical element as defined herein.

A biosensor is analytical device for the detection of a target analyte. A biosensor comprises a bioreceptor element (molecular probe) and a transduction element. The bioreceptor element (molecular probe) can interact with the target analyte to create a physical-chemical change, which is transformed into a measurable signal by the transduction element. The signal is measured by optical spectroscopy such as, but not limited to, infrared ATR spectroscopy, transmission spectroscopy, Raman spectroscopy, colorimetric microscopy, fluorescent microscopy, luminescent microscopy, or combinations of these techniques.

Hence, the optical biosensor according to the invention can be used in conjunction with infrared ATR spectroscopy, transmission spectroscopy, Raman spectroscopy, colorimetric microscopy, fluorescent microscopy, luminescent microscopy, or combinations of these techniques.

According to one preferred embodiment, the device is a transmission IR-based biosensor or an ATR-IR-based biosensor, and more preferably the device is an ATR-IR-based biosensor. Such devices are known in the art, and are for example described in Nabers et al, Anal. Chem. 2016, 88, 2755-2762.

### Process for preparing the surface-functionalized optical element

In one aspect, the invention provides a process for preparing a surface-functionalized optical element according to the invention. The process comprises the steps of:
(a) providing an IR-transparent optical element,
(b) reacting at least a part of the surface of the optical element with a poly(alkylene oxide)-containing compound.

In step a), an IR-transparent optical element is provided. The IR-transparent optical element is preferably an IR-transparent optical element as defined herein above in connection with the surface-functionalized optical element of the invention.

In step b), at least a part of the surface of the IR-transparent optical element is reacted with a poly(alkylene oxide)-containing compound. In step b), the poly(alkylene oxide)-containing compound reacts with the surface of the IR-transparent optical element to create a covalent bond.

In one preferred embodiment, the poly(alkylene oxide)-containing compound is a poly(alkylene oxide)-containing silane or a poly(alkylene oxide)-containing thiol, and more preferably a poly(alkylene oxide)-containing silane. In the latter case, the silane of the poly(alkylene oxide)-containing silane reacts with the surface of the IR-transparent optical element to create a covalent bond.

In one more preferred embodiment, the poly(ethylene oxide)-containing compound is a poly(ethylene oxide)-containing silane or a poly(ethylene oxide)-containing thiol, and even more preferably a poly(ethylene oxide)-containing silane.

It is preferred that the surface of the optical element is first pre-functionalized followed by subsequent attachment of the molecular probe or a linking unit of the linker construct onto the pre-functionalized surface. The process according to the invention can further be defined by one of the synthetic procedures (A) and (B) as defined herein below:

### Synthetic procedure (A)

Synthetic procedure (A) relates to procedures, in which the surface of the IR-transparent optical element is at least partially pre-functionalized by a group selected from nucleophile-reactive groups.

In synthetic procedure (A), the poly(alkylene oxide)-containing compound, preferably the poly(alkylene oxide)-containing silane, comprises a nucleophile-reactive group which is suitable for directly attaching a molecular probe.

According to one embodiment, the poly(alkylene oxide)-containing compound of step b), preferably the poly(alkylene oxide)-containing silane, comprises a group selected from nucleophile-reactive groups selected from aldehydes, carboxylic acids, esters, active esters and maleimide.

Hence, according to one embodiment, an optical element is obtained in step b) having a surface which is at least partially pre-functionalized, and preferably pre-functionalized by a group selected from nucleophile-reactive groups as defined herein above.

Synthetic procedure (A) further comprises a step c) of reacting a molecular probe with the at least partially pre-functionalized surface of the optical element obtained in step b).

For example, in case the molecular probe comprises one or more amine groups, e.g. as part of one or more lysine groups of a peptide/protein, the molecular group can be reacted with a surface of the optical element which has been pre-functionalized by a compound, preferably a silane, comprising a nucleophile-reactive group (e.g. a NHS-ester group).

According to one embodiment, the process comprises the steps:
(a) providing an optical element as defined herein,
(b) reacting at least a part of the surface of the optical element with a poly(alkylene oxide)-containing compound, preferably a poly(alkylene oxide)-containing silane, wherein the compound comprises a group selected from nucleophile-reactive groups, preferably selected from aldehydes, carboxylic acids, esters, active esters and maleimide,
   to obtain an optical element is obtained in step b) having a surface which is at least partially pre-functionalized,
(c) reacting a molecular probe with the at least partially pre-functionalized surface of the optical element obtained in step (c) to obtain a surface-functionalized optical element according to the invention.

### Synthetic procedure (B)

Synthetic procedure (B) relates to procedures, in which the surface of the IR-transparent optical element is at least partially pre-functionalized by a group selected from bioorthogonal-reactive groups. "Bioorthogonal-reactive groups" are known to the skilled person and can be, but are not limited to, alkynes, strained cycloalkynes, strained cycloalkenes, azides, tetrazines, nitrones, norbornenes, nitrile oxides, oxanorbornadienes, and tetrazoles.

"Strained cycloalkynes" are known to the skilled person and include, but are not limited to, 8-membered cycloalkynes. "Strained cycloalkenes" include, but are not limited to, trans-cyclooctene.

In synthetic procedure (B), the poly(alkylene oxide)-containing compound, preferably the poly(alkylene oxide)-containing silane, comprises a bioorthogonal-reactive group which is suitable for attaching a linking unit of the linker construct. Synthetic procedure (B) is preferred.

According to one embodiment, the poly(alkylene oxide)-containing compound of step b), preferably the poly(alkylene oxide)-containing silane, comprises a group selected from bioorthogonal-reactive groups, preferably selected from alkynes, strained cycloalkynes, strained cycloalkenes, azides, and tetrazines.

Hence, according to this embodiment, an optical element is obtained in step b) having a surface which is at least partially pre-functionalized by a group selected from bioorthogonal-reactive groups, preferably selected from alkynes, strained cycloalkynes, strained cycloalkenes, azides, and tetrazines.

According to one embodiment, step b) comprises reacting at least a part of the surface of the optical element with a poly(alkylene oxide)-containing compound according to formula (C-A1)

Y^{a}-L^{a}-X^{a} (C-A1),

wherein
Y^{a} is -SH or -SiZ^{a}₃, wherein each Z^{a} is independently selected from -O-(CH₂)r-CH₃, - (CH₂)t-CH₃, and halogen, wherein r is an integer between 0 to 4, and t is an integer between 0 to 6,
L^{a} is a linking moiety comprising the poly(alkyene oxide), and
X² is -N₃, a cyclo-trans-alkene group, an alkyne group, or 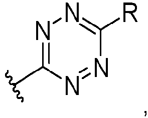 wherein R is an optional substituent, preferably an optional C1-C6 alkyl substituent (e.g. methyl),
and preferably X^{a} is -N₃.

The cyclo-trans-alkene group may be, but is not limited to, an optionally substituted 8-membered cyclo-trans-alkene optionally comprising one or more heteroatoms.

Suitable alkyne groups of substituent X^{a} are alkyne groups comprising a structural unit of DBCO, DIBO, DIFO, or BCN. These abbreviations are well-known in the art. For illustration, DBCO is 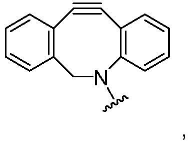 DIBO is 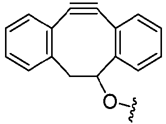 and DIFO is

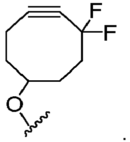

According to one preferred embodiment, step b) comprises reacting at least a part of the surface of the optical element with a poly(alkylene oxide)-containing compound according to formula (C-A1)

Y^{a}-L^{a}-X^{a} (C-A1),

wherein
Y^{a} is -SiZ^{a}₃, wherein each Z^{a} is independently selected from -O-(CH₂)r-CH₃, -(CH₂)t-CH₃, and halogen, wherein r is an integer between 0 to 4, and t is an integer between 0 to 6, and preferably wherein each Z^{a} is -OCH₃ or -OCH₂CH₃,
L^{a} is a linking moiety comprising the poly(alkyene oxide), and
X^{a} is -N₃, a cyclo-trans-alkene group, an alkyne group, or 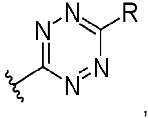 wherein R is an optional substituent, preferably an optional C1-C6 alkyl substituent (e.g. methyl),
and preferably X^{a} is -N₃.

According to one embodiment, step b) comprises reacting at least a part of the surface of the optical element with a poly(alkylene oxide)-containing compound according to formula (C-A2)

Y^{a}-(CH₂)u-X^{a1}-(CH₂)v-X^{a2}-X^{a3}-X^{a} (C-A2),

wherein
Y^{a} is -SH or -SiZ^{a}₃, wherein each Z^{a} is independently selected from -O-(CH₂)r-CH₃, - (CH₂)t-CH₃, and halogen, wherein r is an integer between 0 to 4, and t is an integer between 0 to 6,
u is an integer from 1 to 6;
X^{a1} is a bond, -C(O)NH- or -NHC(O)-;
v is an integer from 1 to 16;
X^{a2} is -(OCH₂CH₂)n¹-, wherein n¹ is an integer from 1 to 16;
X^{a3} is a bond or -CH₂-;
X^{a} is -N₃, a cyclo-trans-alkene group, an alkyne group, or 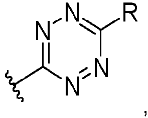 wherein R is an optional substituent, preferably an optional C1-C6 alkyl substituent (e.g. methyl),
and preferably X^{a} is -N₃.

According to one preferred embodiment, step b) comprises reacting at least a part of the surface of the optical element with a poly(alkylene oxide)-containing compound according to formula (C-A2)

Y^{a}-(CH₂)u-X^{a1}-(CH₂)v-X^{a2}-X^{a3}-X^{a} (C-A2),

wherein
Y^{a} is -SiZ^{a}₃, wherein each Z^{a} is independently selected from -O-(CH₂)r-CH₃, -(CH₂)t-CH₃, and halogen, wherein r is an integer between 0 to 4, and t is an integer between 0 to 6, and preferably wherein each Z^{a} is -OCH₃ or -OCH₂CH₃,
u is an integer from 1 to 6;
X^{a1} is a bond, -C(O)NH- or -NHC(O)-;
v is an integer from 1 to 16;
X^{a2} is a bond or -(OCH₂CH₂)n¹-, wherein n¹ is an integer from 1 to 16;
X^{a3} is a bond or -CH₂-;
X^{a} is -N₃, a cyclo-trans-alkene group, an alkyne group, or 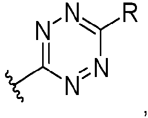 wherein R is an optional substituent, preferably an optional C1-C6 alkyl substituent (e.g. methyl),
and preferably X^{a} is -N₃.

In a specific embodiment, the poly(alkylene oxide)-containing compound of step b) can have the following structure:

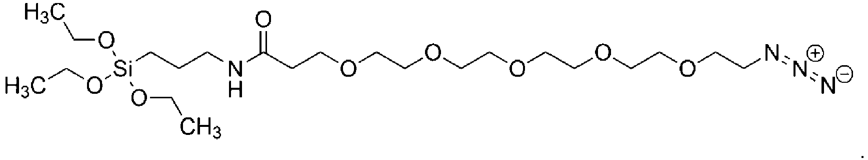

The pre-functionalized optical element can subsequently be reacted in a step c) with a molecular probe (*Synthetic procedure (B1)*) or a pre-labeled molecular probe (*Synthetic procedure* (B2)) to obtain the inventive surface-functionalized optical element.

Alternatively, the pre-functionalized optical element can subsequently be reacted with a linking unit of the linker construct to further prepare the surface of the optical element for attachment of the molecular probe (*Synthetic procedure (B3)*)*.*

### Synthetic procedure (B1)

In synthetic procedure (B1), the pre-functionalized optical element obtained in step (b) of Synthetic procedure (B) is subsequently reacted in a step (c) with a molecular probe which comprises one or more bioorthogonal reactive groups, e.g. an azide as part of an unnatural amino acid of a peptide/protein. In step (c), the surface-functionalized optical element according to the invention is obtained.

The molecular probe reacted in step (c) comprises one or more bioorthogonal reactive-group which is suitable for reacting with a bioorthogonal reactive group of the pre-functionalized surface of the optical element. Such pairs of bioorthogonal reactive groups are known to the skilled person and include, but are not limited to, azides/alkynes, azides/strained alkynes, trans-alkenes/tetrazenes.

### Synthetic procedure (B2)

In synthetic procedure (B2), the pre-functionalized optical element obtained in step (b) of Synthetic procedure (B) is subsequently reacted in a step (c) with a pre-labeled molecular probe. In step (c), the surface-functionalized optical element according to the invention is obtained.

Hence, according to one embodiment, the process comprises a step (c) of reacting the pre-functionalized surface of the optical element with a pre-labeled molecular probe. It is preferred that the pre-labeled molecular probe is pre-labeled with a bioorthogonal reactive-group which is suitable for reacting with a bioorthogonal reactive group of the pre-functionalized surface of the optical element. Such pairs of bioorthogonal reactive groups are known to the skilled person and include, but are not limited to, azides/alkynes, azides/strained alkynes, trans-alkenes/tetrazenes.

The bioorthogonal reactive group can be covalently attached to the molecular probe by a linking unit. For example, the pre-labeling of the molecular probe may be carried out by reacting the molecular probe with a labeling compound comprising (i) a bioorthogonal reactive group, (ii) a nucleophile reactive group (e.g. NHS active ester), and wherein the nucleophile reactive group reacts with the molecular probe.

According to one embodiment, the pre-labeled molecular probe comprises an alkyne group, and optionally an alkyne group comprising a structural unit of DBCO, DIBO, DIFO, or BCN.

### Synthetic procedure (B3)

In synthetic procedure (B3), the pre-functionalized optical element obtained in step (b) is not directly reacted to obtain the inventive surface-functionalized optical element in a single step (c), but step (c) can comprise one or more of additional steps (c-1) to (c-5).

In synthetic procedure (B3), the pre-functionalized optical element obtained in step (b) of Synthetic procedure (B) is subsequently reacted in a step (c-1) with a linking unit precursor comprising (i) a bioorthogonal reactive-group which is suitable for reacting with a bioorthogonal reactive group of the pre-functionalized surface of the optical element, and (ii) a nucleophile reactive group as defined herein.

The bioorthogonal reactive group (i) of the linking unit precursor is preferably selected from azide, an alkyne group, a trans-alkene group, and a tetrazene. The nucleophile-reactive group (ii) of the linking unit precursor is preferably selected from aldehydes, carboxylic acids, esters, active esters and maleimide, and more preferably is a NHS-active ester or a TFP-active ester.

In one specific example, the linking unit precursor used in step (c-1) has the following structure:

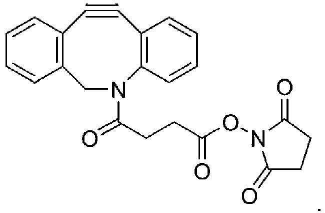

In step (c-1), an optical element is obtained having a surface which is at least partially pre-functionalized by a nucleophile-reactive group.

The pre-functionalized optical element obtained in step (c-1) can subsequently be used in a step (c-2) of the process to attach a molecular probe. Thus, according to one embodiment, the process comprises a step (c-2) of reacting a molecular probe with the optical element obtained in step (c-1) to obtain the surface-functionalized optical element according to the invention. The attachment in step (c-2) is achieved by reacting the molecular probe (e.g. a H₂N-group of a lysine side chain of the molecular probe) with the nucleophile reactive group of the optical element obtained in step (c-1).

Alternatively, the pre-functionalized optical element obtained in step (c-1) can subsequently be used in a step (c-3) of the process to attach the peptide compound of the peptide moiety. The attachment in step (c-3) is achieved by reacting the molecular probe or the peptide compound with the nucleophile reactive group of the optical element obtained in step (c-1).

According to another embodiment, the process comprises a step (c-3) of reacting a peptide compound with the optical element obtained in step (c-1) to obtain an optical element having a surface which is at least partially functionalized by a peptide compound. The peptide-functionalized optical element obtained in step (c-3) can be understood in that at least a part of the surface of the optical element has a covalently bound peptide layer. This peptide layer can have the function of a blocking layer in the final surface-functionalized optical element.

The peptide compound of the peptide-functionalized optical element obtained in step (c-3) can subsequently be reacted in a step (c-4) with a second linking unit precursor. The second linking unit precursor can be any precursor which is suitable for attaching a molecular probe (pre-labeled or non-pre-labeled) to the peptide-functionalized surface. Step (c-4) provides a pre-functionalized surface which can subsequently be reacted in a step (c-5) with the molecular probe to provide the inventive surface-functionalized optical element.

Typically, the second linking unit precursor will have two reactive groups, of which one reactive group is used to attach the second linking unit precursor to the peptide compound and the other reactive group is used as attachment point for the molecular probe.

According to one embodiment, the process comprises a step (c-4) of reacting a linker precursor B comprising (i) a bioorthogonal reactive-group, and (ii) a nucleophile reactive group. The bioorthogonal reactive group (i) and the nucleophile reactive group (ii) of the second linking unit precursor can be selected from the groups defined herein above for the first linking unit precursor.

In this embodiment of step (c-4), the peptide compound of the optical element obtained in step (c-3) are reacted with the nucleophile reactive group of the second linking unit precursor. In particular, the N-terminus and/or reactive side chains (e.g. lysine side chain, serine side chain, cysteine side chain) of the peptide compound can react in this embodiment of step (c-4) with the nucleophile reactive group of the second linking unit precursor.

According to one specific embodiment, the second linking unit precursor has the following structure:

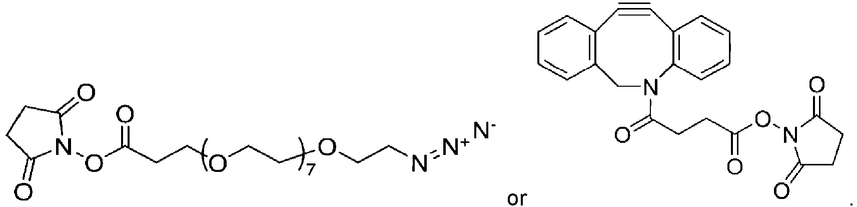

In step (c-4), an optical element is obtained, wherein the optical element comprises a pre-functionalized peptide compound on at least a part of the surface of the optical element.

In a subsequent step (c-5) of the process, the optical element obtained in step (c-4) is reacted with a molecular probe or with a pre-labeled molecular probe. In step (c-5), the surface-functionalized optical element according to the invention is obtained.

According to one embodiment, the process comprises a step (c-5) of reacting the optical element obtained in step (c-4) with a pre-labeled molecular probe. It is preferred that the pre-labeled molecular probe is pre-labeled with a bioorthogonal reactive-group which is suitable for reacting with a bioorthogonal reactive group of the optical element obtained in step (e). Such pairs of bioorthogonal reactive groups are known to the skilled person and include, but are not limited to, azides/alkynes, azides/strained alkynes, trans-alkenes/tetrazenes.

Other options of attaching a molecular probe or a pre-labeled molecular probe on the peptide-functionalized surface obtained in step (c-3) exist and can be selected by the person of skill.

### Method for detecting a target analyte

In another aspect of the invention, a method for detecting a biomarker is provided. The method comprises the steps of
(i) bringing into contact a surface-functionalized optical element as defined herein with a sample suspected to comprise a target analyte, and optionally wherein the sample is a human body fluid;
(ii) detecting the biomarker based on an interaction between the molecular probe and the target analyte, and optionally wherein the interaction between the molecular probe and the target analyte is detected by infrared spectroscopy, and optionally wherein the biomarker is based on an amide I band position of the target analyte.

The sample may be a body fluid such as, but not limited to, blood plasma or cerebrospinal fluid (CSF) sample. According to one embodiment, the sample is a blood plasma or CSF sample. The sample may be brought into contact with the surface-functionalized optical element in a flow chamber.

According to one embodiment, the target analyte may be selected from the group consisting of amyloid-beta (Aβ) peptides, alpha synuclein, tau protein, TDP-43, human islet amyloid polypeptide (hiAPP), prion protein, and p53, and optionally from the group consisting of amyloid-beta (Aβ) peptides, alpha synuclein, tau protein, and TDP-43 . The amyloid-beta (Aβ) peptides may be monomeric, oligomeric or fibrillar amyloid-beta (Aβ) peptides.

The interaction between the molecular probe and the target analyte may be detected by any suitable means detecting a biochemical interaction. The interaction may be detected by a spectroscopical method such as, but not limited to, infrared spectroscopy, UV/Vis spectroscopy, fluorescence spectroscopy, and the like.

According to one embodiment, the interaction between the molecular probe and the target analyte is detected by infrared spectroscopy, and preferably ATR-IR spectroscopy.

According to one embodiment, the interaction between the molecular probe and the target analyte is detected by infrared spectroscopy, and preferably by ATR-IR spectroscopy, and wherein the biomarker is an amide band maximum of the target analyte. The amide I band position may be an amide I band maximum of an amyloid-beta peptide, and is optionally in the range of 1600 to 1700 cm⁻¹, optionally 1620 to 1670 cm⁻¹, and optionally 1640 to 1647 cm⁻¹.

Such methods are known in the art, and are for example described in Nabers et al, Anal. Chem. 2016, 88, 2755-2762.

### Use of the surface-functionalized optical element

In one aspect, the present invention provides a use of surface-functionalized optical element according to the invention or of an optical biosensor according to the invention for one or more of:
a companion diagnostic test,
diagnosing a protein misfolding disease, diabetes or a tumor in a patient,
monitoring of therapy of patients having a protein misfolding disease, diabetes or a tumor, and
for screening of drugs for the treatment of a protein misfolding disease, diabetes or a tumor.

The protein misfolding disease may be one or more of Alzheimer's disease, Parkinson's disease, amyotrophic lateral sclerosis, Huntington, or Prion disease.

According to one embodiment, a use of surface-functionalized optical element as defined herein or of an optical biosensor as defined herein is provided for one or more of:
diagnosing Alzheimer's disease, Parkinson's disease or amyotrophic lateral sclerosis in a patient,
monitoring of therapy of patients with Alzheimer's disease, Parkinson's disease or amyotrophic lateral sclerosis, and
screening of drugs for the treatment of Alzheimer's disease, Parkinson's disease or amyotrophic lateral sclerosis.

According to one embodiment, the surface-functionalized optical element or the optical biosensor is used for diagnosing Alzheimer's disease and/or for monitoring of therapy of patients with Alzheimer's disease.

In one embodiment, a surface-functionalized optical element as defined herein or an optical biosensor as defined herein is provided for use in one or more of:
a companion diagnostic test,
diagnosing a protein misfolding disease, diabetes or a tumor in a patient,
monitoring of therapy of patients having a protein misfolding disease, diabetes or a tumor, and
for screening of drugs for the treatment of a protein misfolding disease, diabetes or a tumor.

### Figures

Fig. 1: ATR-FTIR spectra of NHS-functionalized optical elements in accordance with inventive example 1 (grey: "NHS-PEG-Silane") and comparative example 1 (black: "NHS-Silane").
Fig. 2: Quantity of Antibody based on amine II absorbance during stability tests for inventive example 1 (graph with circles: "Antibody // NHS-PEG-silane") and comparative example 1 (graph with squares: "Antibody // NHS silane"). The figure shows quantity of antibody on the surface for (from left to right): antibody attachment, washing step 1 and washing step 2.
Fig. 3A: Kinetic evaluation of normalized amide II absorbance during stability tests as described in example 2C. The figure shows quantity of peptide compound on the surface for (from left to right): peptide attachment, washing step 1 and washing step 2.
Fig. 3B: Kinetic evaluation of normalized amide II absorbance during washing step 1 as described in example 2C.
Fig. 3C: ATR-FTIR spectra within amide I and II region after washing step 2 as described in example 2C.
Fig. 4: Normalized ATR-FTIR spectra of non-specific protein (NSP) adsorption of BSA described in example 2D.
Fig. 5: Normalized ATR-FTIR spectra of non-specific protein (NSP) adsorption of blood plasma described in example 2D.

In the following, the invention is further described by specific examples. The examples shall not be understood or construed as limiting the invention in any way.

### Examples

### Example 1: Surface-functionalized optical element comprising a linker construct without a peptide moiety

### A. Preparation of the surface-functionalized optical element according to one embodiment of the invention

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Linker I: | 1-azido-N-(3-(triethoxysilyl)propyl)-3,6,9, 12, 15-pentaoxaoctadecan-18-amide |
| Linker II: | 2,5-dioxopyrrolidin-1-yl 6-{2-azatricyclo[10.4.0.0^{4,9}]hexadeca-4,6,8,12,14,16-hexaen-10-yn-2-yl}-6-oxohexanoate |
| Molecular probe: | anti-β-Amyloid (13-28) antibody, mouse monoclonal |
| Device: | ATR-IR-biosensor |

### Pretreatment of the silicon crystal

The surface was activated by an oxidative treatment (plasma treatment) to produce a stable silicon dioxide layer.

### Silanization of a silicon crystal

For silanization of the silicon crystal, a solution of the linker precursor I was prepared in acetone and the activated surface of the silicon crystal was coated by contacting the selected surface of the crystal with the silanization solution. The crystal was rinsed with acetone, followed by a rinse with H₂O, and was subsequently dried under nitrogen.

### NHS activation of the azide moieties of the silanized surface via SPAAC using linker precursor II

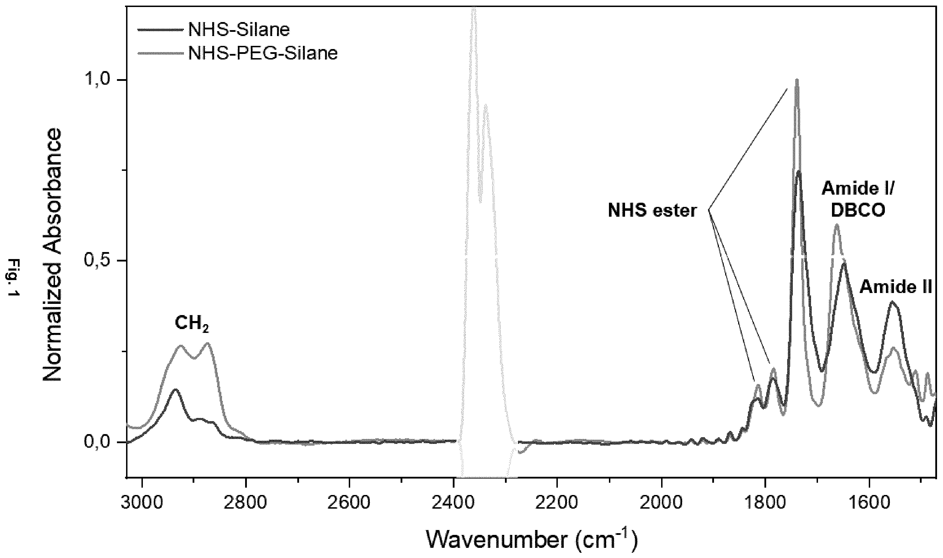

The crystal surface previously silanized with linker precursor I was further reacted with linker precursor II in a SPAAC click reaction to obtain NHS reactivity. As understood by those skilled in the art, the coupling takes place by reaction of the DBCO group with the azide group on the silanized surface. After SPAAC click reaction, reactive NHS groups are available on the silanized silicon crystal which can be reacted with the β-Amyloid (13-28) antibody. After the chemical surface modification of the silicon crystal with linker precursor I and II was complete, the ATR-FTIR difference spectrum showed characteristic NHS ester bands at 1814, 1783 and 1738 cm⁻¹ (Fig. 1, grey spectrum). The combined linker construct from linker precursor I and II is termed in Fig. 1 as "NHS-PEG-Silane".

### Attaching the β-Amyloid (13-28) antibody to the NHS-functionalized surface

The β-amyloid (13-28) antibody was immobilized in low-salt buffer conditions and in flow through mode applying a continuous flow using the ATR-IR-biosensor device. During the attachment process, the NHS-activated surface reacted with primary amines from the antibody, releasing NHS and forming a covalent bond between surface and molecular probe.

The NHS-functionalized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with attachment buffer. Subsequently the β-amyloid (13-28) antibody was circulated over the crystal surface.

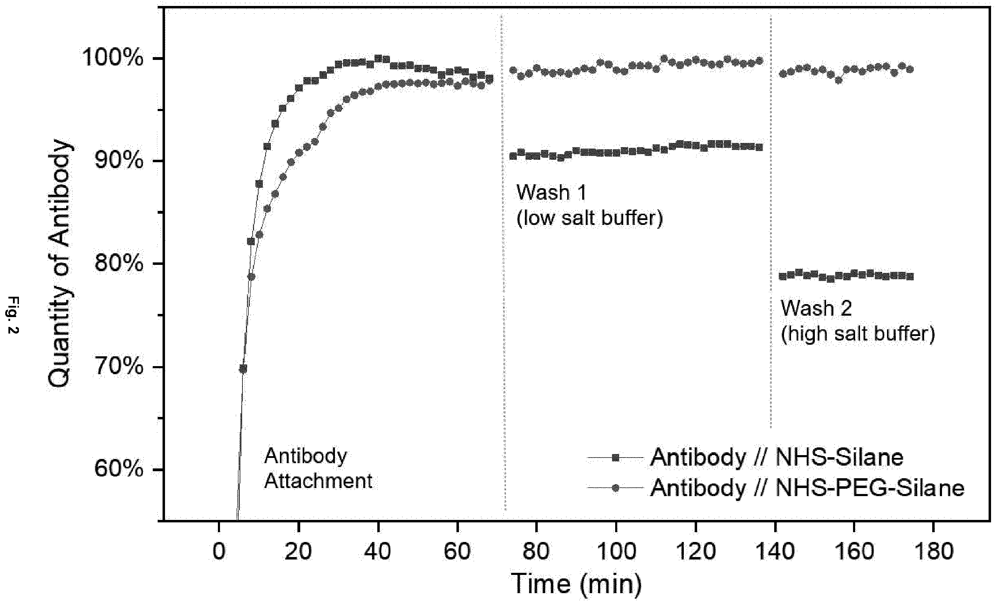

Complete IR spectra were continuously recorded and compared with the background before antibody attachment. An indication for successful immobilization of the antibody on the surface is the amide 2 signal at 1550 cm⁻¹ (Fig. 2, graph with circles "Antibody // NHS-PEG-Silane", part: antibody attachment).

### B. Preparation of comparative surface-functionalized optical element

A comparative surface-functionalized optical element was prepared using a one-step method for preparing the linker construct. A single linker precursor was used which does not contain a poly(alkylene oxide) group.

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Linker precursor III: | N-(4,4,4-triethoxysilanebutyl)succinamic acid 2,5-dioxopyrrolidin-1-yl ester |
| Molecular probe: | anti-β-Amyloid (13-28) antibody, mouse monoclonal |
| Device: | ATR-IR-biosensor |

### Pretreatment of the silicon crystal

The surface was activated by an oxidative treatment (plasma treatment) to produce a stable silicon dioxide layer.

### Silanization of a silicon crystal

For silanization of the silicon crystal, a solution of linker precursor III was prepared in acetone and the activated surface of the silicon crystal was coated by contacting the selected surface of the crystal with the silanization solution. The crystal was rinsed with acetone, followed by a rinse with H₂O, and subsequently dried under nitrogen. After the silanization process with linker III was completed, the ATR-FTIR difference spectrum showed characteristic NHS ester bands at 1814, 1783 and 1738 cm⁻¹ (Fig. 1, black spectrum). The linker construct derived from linker precursor III is termed in Fig. 1 as "NHS-Silane".

### Attaching the β-amyloid (13-28) antibody to the NHS-functionalized surface

The β-amyloid (13-28) antibody was immobilized in low-salt buffer conditions and in flow through mode applying a continuous flow using the ATR-IR-biosensor device. During the attachment process, the NHS-activated surface reacted with primary amines from the antibody, releasing NHS and forming a covalent bond between surface and molecular probe.

The NHS-functionalized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with attachment buffer. Subsequently the β-amyloid (13-28) antibody was circulated over the crystal surface.

Complete IR spectra were continuously recorded and compared with the background before antibody attachment. An indication for successful immobilization of the antibody on the surface is the amide 2 signal at 1550 cm⁻¹ (Fig. 2, graph with squares "Antibody // NHS-Silane"; part: antibody attachment).

### C. Stability test

Stability tests of the molecular probe were performed on the inventive surface-functionalized optical element and on the comparative surface-functionalized optical element. The two surface-functionalized optical elements were prepared as described above under Example 1A and 1B.

Results are shown in Figure 2, wherein the attachment process and wash of the β-amyloid (13-28) antibody is presented in a kinetic profile for both surfaces. The graph with circles shown in Fig. 2 corresponds to the example in accordance with one embodiment of the invention, which contains a poly(alkylene oxide) group in the spacer moiety of its linker construct. The graph with squares corresponds to the comparative example which does not contain a poly(alkylene oxide) group in the linker construct. After the antibody attachment was completed, the maximum of amide 2 absorbance was used for normalization, thus defining the maximum quantity of attached antibody as comparison for the subsequent wash steps. Two wash steps were performed. The first wash step was performed in low salt buffer conditions, while high salt buffer conditions were used in the second wash step. The wash spectra were recorded in attachment buffer conditions, therefore eliminating wash buffer-induced artefacts in the IR spectra.

The kinetic profile in this stability test reveals that a clear difference between the inventive example and the comparative example can be observed. No decrease in the quantity of attached antibody can be monitored for the inventive example. This is indicated by a stable profile of amide 2 absorbance which represents a robust antibody immobilization.

In contrast, the comparative example shows a clear loss of attached antibody in both wash steps. After the first wash step in low salt buffer conditions, around 90% of antibody remain and the amount of antibody further decreases to about 80% in the second wash step, where high salt buffer conditions were applied.

When recording the respective wash spectra in attachment buffer, a stable profile of amide 2 absorbance can be observed in between the wash steps on both surfaces. This shows that the comparative example may be stable in the attachment buffer. However, the inventive example demonstrates a high robustness under all conditions, including challenging high salt buffer conditions.

The stability tests show that the use of a spacer moiety comprising a poly(alkylene oxide) improves the stability of the surface-functionalized optical element compared to a linker construct which does not contain a poly(alkylene oxide) as demonstrated by the comparative example.

### Example 2: Surface-functionalized optical element comprising a linker construct with a peptide moiety

### A. Preparation of a surface-functionalized optical element comprising a linker construct with a peptide moiety (linker construct as defined herein)

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Linker precursor I: | 1-azido-N-(3-(triethoxysilyl)propyl)-3,6,9, 12, 15-pentaoxaoctadecan-18-amide |
| Linker precursor II: | 2,5-dioxopyrrolidin-1-yl 6-{2-azatricyclo[10.4.0.0^{4,9}]hexadeca-4,6,8,12,14,16-hexaen-10-yn-2-yl}-6-oxohexanoate |
| Peptide compound: | casein fragment blocking solution, made from bovine casein (Sigma Aldrich / Art.-Nr.: C3400) by alkaline hydrolysis |
| Device: | ATR-IR-biosensor |

### Pretreatment, silanization and NHS-activation of the silicon crystal

Pretreatment, silanization with linker precursor I and NHS-activation with linker precursor II were carried out as described above for inventive example 1.

### Functionalization of surface with peptide moieties derived from a casein-based blocking solution

After the silicon surface was silanized and NHS-activated with linker precursor I and II, a protein-reactive silicon crystal was available. In the subsequent surface functionalization, the NHS-functionalized crystal surface was further functionalized with peptide moieties from a casein-based blocking solution. Covalent bonding can be achieved via amide bonding of the primary amine group of a lysine of the casein peptides or the N-termini of the casein peptides with the NHS-functional groups of the silicon crystal.

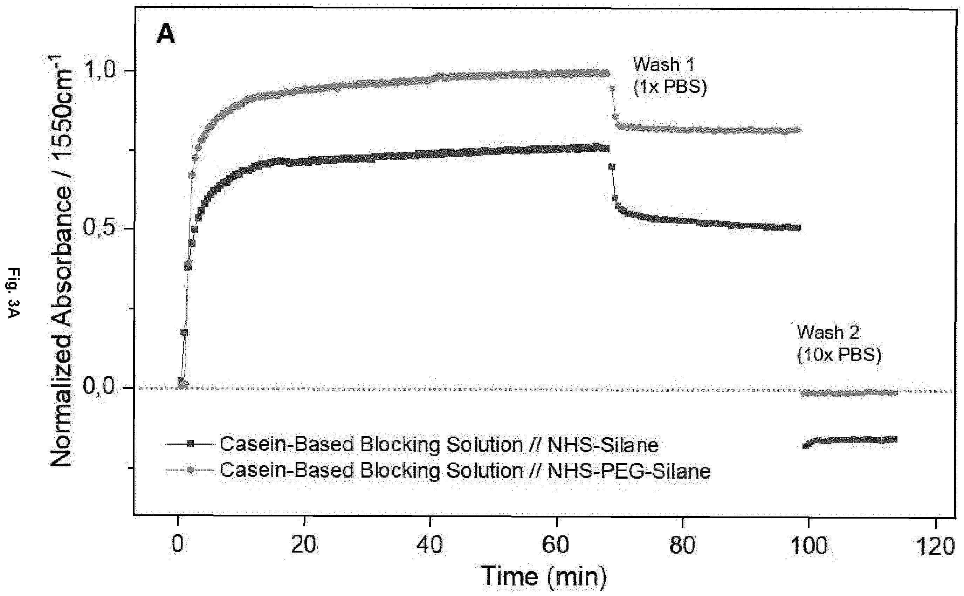

The NHS-functionalized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with PBS buffer. Subsequently the casein fragment blocking solution was circulated over the crystal surface, followed by a washing step with PBS in flow-through mode. During the functionalization of the surface with peptide moieties, complete IR spectra were continuously recorded and referenced against the PBS background. The process was monitored by following an increase of the amide 2 absorbance at 1550 cm⁻¹ over time. Figure 3A (red dots) shows the corresponding kinetic profile of attachment and wash of the casein-based peptide solution on the inventive surface.

Next, excess NHS groups were quenched with an ethanolamine solution followed by another washing step.

The functionalized surface prepared in this example 2A corresponds to a linker construct comprising a peptide moiety as defined herein. The functionalized surface prepared in this example can subsequently be further functionalized with a molecular probe as defined herein, e.g. under Synthetic procedure (B3), steps (c-3) to (c-5) herein above, or in Example 3 below.

### B. Preparation of a comparative surface-functionalized optical element comprising a peptide moiety (linker w/o poly(alkylene oxide) group)

A comparative surface-functionalized optical element was prepared using a one-step method for preparing the linker construct. A single linker precursor was used which does not contain a poly(alkylene oxide) group.

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Linker precursor III: | N-(4,4,4-triethoxysilanebutyl)succinamic acid 2,5-dioxopyrrolidin-1-yl ester |
| Peptide compound: | casein fragment blocking solution, made from bovine casein (Sigma Aldrich / Art.-Nr.: C3400) by alkaline hydrolysis |
| Device: | ATR-IR-biosensor |

### Pretreatment and silanization of the silicon crystal

Pre-treatment and silanization of the silicon crystal were carried out as described in comparative example 1 above.

### Functionalization of surface with peptide moieties derived from a casein-based blocking solution

After the silicon surface was silanized and NHS-activated with linker B, a protein-reactive silicon crystal was available. In the subsequent surface functionalization, the NHS-functionalized crystal surface was further functionalized with peptide moieties from a casein-based blocking solution.

The NHS-functionalized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with PBS buffer. Subsequently the casein fragment blocking solution was circulated over the crystal surface, followed by a washing step with PBS in flow-through mode.

During the functionalization of the surface with peptide moieties, complete IR spectra were continuously recorded and referenced against the PBS background. The process was monitored by following an increase of the amide 2 absorbance at 1550 cm⁻¹ over time. Figure 3A shows (grey boxes) the corresponding kinetic profile of attachment and wash of the casein-based peptide solution on the comparative surface.

Next, excess NHS groups were quenched with an ethanolamine solution followed by another washing step.

### C. Stability test

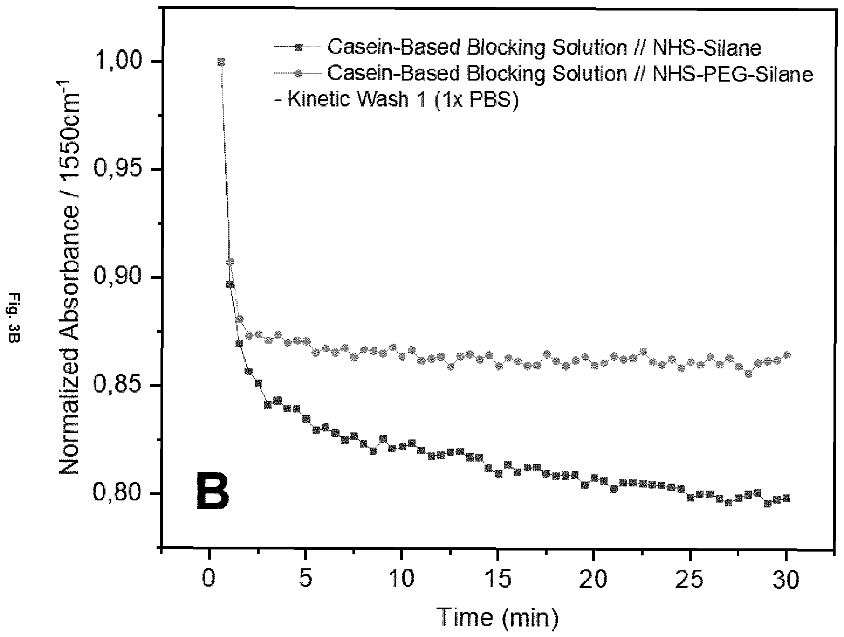

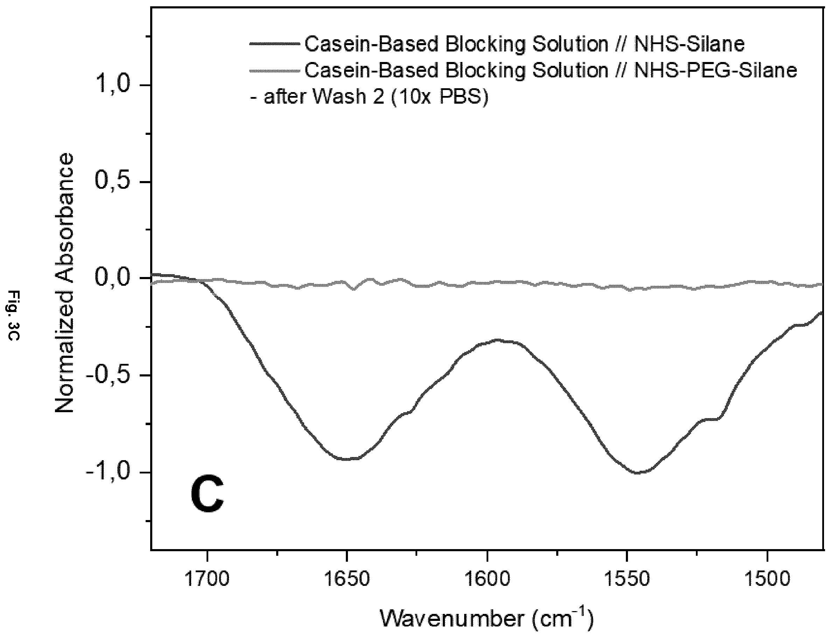

Stability tests with the surface-functionalized optical elements as described in Example 2A and 2B were carried out. Results are shown in Figure 3A to 3C. The attachment and wash of a casein-based blocking solution is presented in a kinetic profile for the functional surfaces of examples 2A and 2B in Fig. 3A and 3B and an additional IR spectrum is shown for the second wash in Fig. 3C. The functional surface according to Example 2A, which comprises the poly(alkylene oxide) group in the spacer moiety of its linker construct, is depicted in the graph with circles in Fig. 3A and referred to as "Casein-Based blocking solution // NHS-PEG-Silane". The comparative functional surface according to Example 2B is depicted by the graph with squares in Fig. 3A and referred to as "Casein-Based blocking solution // NHS-Silane".

The attachment process of the casein-derived peptides was performed for 70 minutes. The functional surfaces according to Examples 2A and 2B both comprise an appreciable amount of attached peptide moieties. However, as can be seen from the data in Fig. 3A, the functional surface according to Example 2A accumulates about 25% more peptide moieties compared to the functional surface according Example 2B.

This result was confirmed in a first wash step with PBS buffer. In the washing step, the functional surfaces according to Examples 2A and 2B first lose similar amounts of loosely attached peptides. After the functional surfaces were rinsed for 30 minutes, the functional surface according to example 2A shows about 40% more surface-bound peptide compared to example 2B. This increase in percentage results from different wash kinetics (Fig. 3B). The functional surface according to example 2A shows a more stable kinetic profile as indicated by the steady amide 2 absorbance, demonstrating a more robust peptide-modified linker construct than the functional surface according to example 2B.

Surface stability of the peptide-modified linker construct was further tested in a high salt buffer washing step using a PBS tenfold buffer solution for 10 minutes in flow-through mode. After that IR spectra were recorded in normal PBS conditions to avoid buffer-related artefacts in the spectrum (Fig. 3C). The high salt buffer wash demonstrated an improved robustness and stability of the functional surface according to example 2A compared to example 2B. No negative absorbance bands were observed in the amide 1 and 2 regions for example 2A. The effect of high salt buffer conditions was different on the functional surface of example 2B, where negative amide bands visualized a detachment of the peptide-modified linker construct.

The stability tests provided herein for the functional surfaces according to examples 2A and 2B show that the use of a spacer moiety comprising a poly(alkylene oxide) improves the stability of the surface-functionalized optical element compared to a linker construct which does not contain a poly(alkylene oxide).

### D. Inertness test

Functional surfaces prepared according to examples 2A and 2B were subjected to an inertness test demonstrating the susceptibility of the two surfaces in terms of non-specific protein (NSP) adsorption. The surfaces were tested for NSP adsorption using bovine serum albumin (BSA) and human blood plasma.

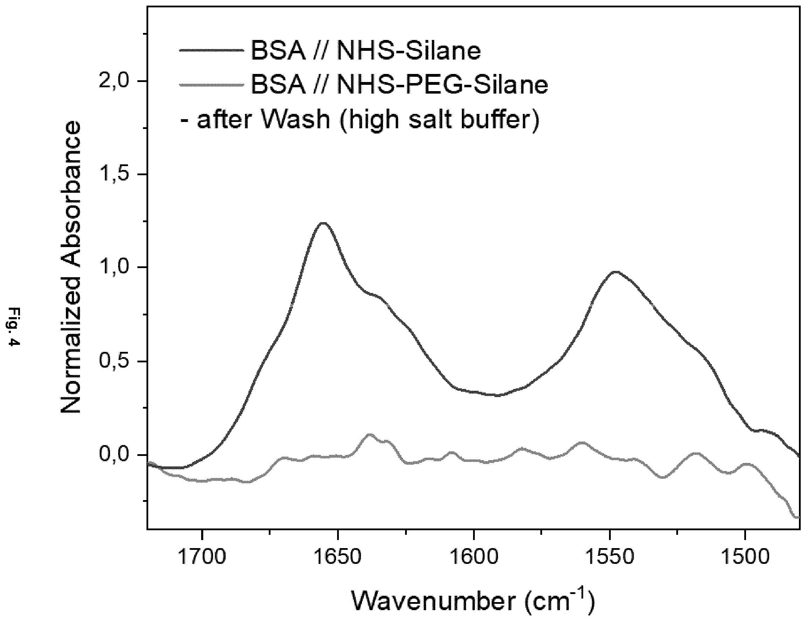

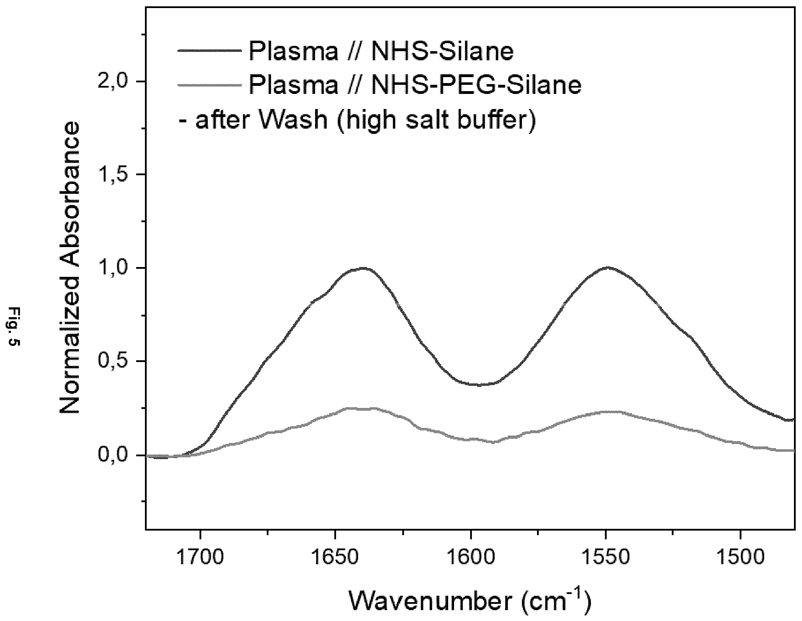

Figure 4 shows the result after BSA was circulated for 60 minutes over the functional surface prepared according to example 2A and 2B, respectively, and washed for another 60 minutes with a tenfold PBS in flow-through mode. The functional surface according to example 2A is referred to as "BSA // NHS-PEG-Silane" (lower graph in Fig. 4 and 5). The comparative functional surface according to example 2B is referred to as "BSA // NHS-Silane" (upper graph in Fig. 4 and 5).

During both steps, IR spectra were continuously recorded, revealing significantly improved properties with respect to NSP adsorption on the functional surface of example 2A. For BSA, no non-specific attachment was observed on the functional surface prepared in accordance with example 2A, whereas a NSP adsorption of BSA was present on the functional surface prepared in accordance with example 2B. This result was confirmed to a certain extent when human blood plasma was circulated for 60 minutes and washed with a tenfold PBS for another 60 minutes in flow-through mode. Results of NSP adsorption after plasma treatment are shown in figure 5, demonstrating the reduction of NSP adsorption from plasma by a factor of 5 for the functional surface of example 2A compared to the comparative surface of example 2B. The position of the amide 1 band maximum as well as the overall shape are similar on both surfaces, indicating that there is no different in the secondary structure distribution of the non-specifically bound proteins.

The inertness tests provided herein for the functional surfaces according to examples 2A and 2B show that the use of a spacer moiety comprising a poly(alkylene oxide) improves inertness towards non-specific binding of proteins and human blood plasma compared to a comparable functionalized surface which does not contain such a spacer moiety in its linker construct. An improved inertness in turn can improve a detection of a target analyte from a complex sample.

Example 2 further indicates that a surface-functionalized optical element according to one embodiment of the invention has an improved stability and inertness, and therefore can advantageously be used as part of a biosensor as described herein.

### Example 3: Surface-functionalized optical element comprising a linker construct with a peptide moiety according to one embodiment of the invention

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Linker precursor I: | 1-azido-N-(3-(triethoxysilyl)propyl)-3,6,9,12,15-pentaoxaoctadecan-18-amide |
| Matrix precursor compound: | 3-(2-(2-methoxyethoxy)ethoxy)-N-(3-(triethoxysilyl)propyl)propanamide |
| Linker precursor II: | 2,5-dioxopyrrolidin-1-yl 6-{2-azatricyclo[10.4.0.0^{4,9}]hexadeca-4,6,8,12,14,16-hexaen-10-yn-2-yl}-6-oxohexanoate |
| Peptide compound: | casein fragment blocking solution, made from bovine casein (Sigma Aldrich / Art.-Nr.: C3400) by alkaline hydrolysis |
| Linker precursor IV: | 2,5-dioxopyrrolidin-1-yl 1-{2-azatricyclo[10.4.0.0^{4,9}]hexadeca-4,6,8,12,14,16-hexaen-10-yn-2-yl}-1,4-dioxo-7,10,13,16,19,22,25,28,31,34,37,40,43-tridecaoxa-3-aza-hexatetracontan-46-oate |
| Linker precursor V: | 2,5-dioxopyrrolidin-1-yl 1-azido-3,6,9,12,15,18,21,24-octaoxaheptacosan-27-oate |
| Molecular probe: | anti-β-Amyloid (13-28) antibody, mouse monoclonal |
| Device: | ATR-IR-biosensor |

### Pretreatment of the silicon crystal

The surface was activated by an oxidative treatment to produce a stable silicon dioxide layer.

### Silanization of a silicon crystal

For silanization of the silicon crystal, a mixture of linker precursor I and the matrix precursor compound in 2-propanol was prepared and contacted with a selected surface of the silicon crystal. The crystal was subsequently rinsed with H₂O and dried under nitrogen. After completion of the silanization, the ATR-FTIR difference spectrum (before and after silanization) shows an azide band at 2110 cm⁻¹.

### NHS activation of the azide moieties of the silanized surface

The linker precursor II was reacted in a SPAAC click reaction with the previously silanized crystal surface. After completion of the SPAAC click reaction, the ATR-IR difference spectrum (before silanization / after click reaction) showed 3 NHS bands at 1738 cm⁻¹, 1782 cm⁻¹, and 1815 cm⁻¹. The azide band at 2110 cm⁻¹ has disappeared in the difference spectrum.

### Production of azide-labeled antibody

The anti-amyloid-beta antibody was reacted with the linker precursor V for preparing an azide-labeled anti-amyloid-beta antibody.

### Blocking of surface and immobilization of antibody on blocking layer

After silanization and NHS-activation by the SPAAC click reaction, a protein-reactive silicon crystal was available. In the subsequent surface functionalization, the NHS-functionalized crystal surface was first blocked with the casein fragment solution. The NHS-functionalized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with PBS buffer. Subsequently the casein fragment blocking solution was circulated over the crystal surface, followed by a washing step with PBS in flow-through mode. During the blocking process, complete IR spectra were continuously recorded and referenced against the PBS background. The blocking process was monitored by following an increase of the amide 2 absorbance (1550 cm-1) and simultaneously a decrease of NHS absorbance (1740 cm⁻¹) in time. Next, excess NHS groups were quenched with an ethanolamine solution followed by another washing step.

Subsequently, the peptide blocking layer was DBCO functionalized by reaction with the NHS- and DBCO-containing linker precursor III in flow-through mode.

In a next step, the previously prepared azide-labeled anti-amyloid-beta antibody was immobilized on the DBCO-functionalized peptide blocking layer by a SPAAC click reaction between the DBCO moiety and the azide group in flow-through mode. During antibody attachment, complete IR spectra were continuously recorded and compared with the background before antibody attachment. An indication for successful immobilization of the antibody on the surface is the amide 2 signal (1550 cm-1).

## Claims

1. A surface-functionalized optical element comprising
an IR-transparent optical element,
wherein at least a part of the surface of the IR-transparent optical element is bound to a molecular probe by a linker construct,
wherein the linker construct comprises
a surface attachment moiety,
a spacer moiety which comprises a poly(alkylene oxide), and
a peptide moiety, which is covalently bound between the spacer moiety and the molecular probe.

2. The surface-functionalized optical element according to claim 1, wherein the linker construct has a structure according to formula (B):
//-(S)-(SP)-(Pep)-\\ (B),
wherein
// represents attachment to the surface of the IR-transparent optical element,
(S) represents the surface attachment moiety,
(SP) represents the spacer moiety,
(Pep) is the peptide moiety comprising a peptide compound which comprises at least two amino acids which are linked by a peptide bond, and
\\ represents attachment to the peptide moiety of the linker construct,
wherein the surface attachment moiety (S) has a structure according to formula (S-I):
//-Y¹-L¹-X¹-$$¹ (S-I),
wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is an optional linking unit,
X¹ is a group selected from -C(O)NH-, -NHC(O)-, -NHC(O)HN-, and -NHC(O)O-,
$$¹ represents attachment to the spacer moiety.

3. The surface-functionalized optical element according to claim 1 or claim 2, wherein the linker construct has a structure according to formula (B):
//-(S)-(SP)-(Pep)-\\ (B),
wherein
// represents attachment to the surface of the IR-transparent optical element,
(S) represents the surface attachment moiety,
(SP) represents the spacer moiety,
(Pep) is the peptide moiety comprising a peptide compound which comprises at least two amino acids which are linked by a peptide bond, and
\\ represents attachment to the peptide moiety of the linker construct,
wherein the spacer moiety (SP) has a structure according to formula (SP-II)
$$²-L²-X²-L³-H¹-L⁴-§§¹ (SP-II),
wherein
$$² represents attachment to the surface attachment moiety (S),
L² to L⁴ is each independently an optional linking unit,
X² is -(OCH₂CH₂)n¹-, wherein n1 is an integer from 2 to 100, preferably from 2 to 50, and more preferably from 2 to 25,
H¹ is an optionally substituted N-heterocyclic group having a structure according to any one of formula (HET-I) to (HET-VI): 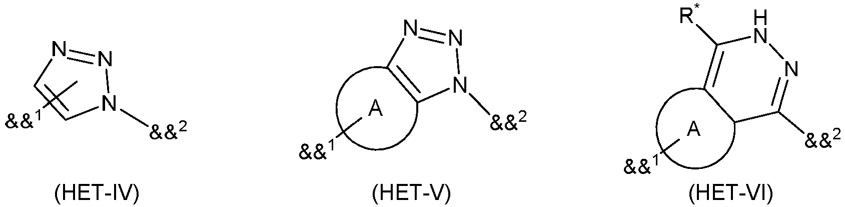 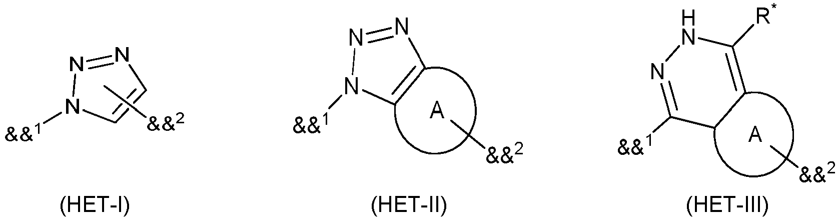
wherein
&&¹ represents attachment to L³ or, if L³ is absent, corresponds to X²,
&&² represents the attachment to L⁴ or, if L⁴ is absent, corresponds to §§¹,
R* is H, an optionally substituted alkyl group or an optionally substituted aryl group, and preferably is a C1-C6 alkyl group,
ring A is an optionally substituted 8-membered carbocycle or an optionally substituted 8-membered heterocycle, and is preferably an optionally substituted dibenzo-fused 8-membered N-heterocycle;
§§¹ represents the attachment to the peptide moiety of the linker construct.

4. The surface-functionalized optical element according to any one of claims 1 to 3, wherein the linker construct has a structure according to formula (B):
//-(S)-(SP)-(Pep)-\\ (B),
wherein
// represents attachment to the surface of the IR-transparent optical element,
(S) represents the surface attachment moiety,
(SP) represents the spacer moiety,
(Pep) is the peptide moiety,
\\ represents attachment to the molecular probe,
and wherein the peptide moiety (Pep) has a structure according to Formula (PEP-I):
§§²-P¹-L⁵-H²-L⁶-##¹ (PEP-I),
wherein
§§² represents attachment to the spacer moiety (SP),
P¹ is a peptide compound which is selected from the group consisting of peptides, proteins, protein fragments, each of which is optionally substituted,
L⁵ and L⁶ is each independently an optional linking unit,
H² is an optionally substituted N-heterocyclic group having a structure according to any one of formula (HET-VII) to (HET-XII): 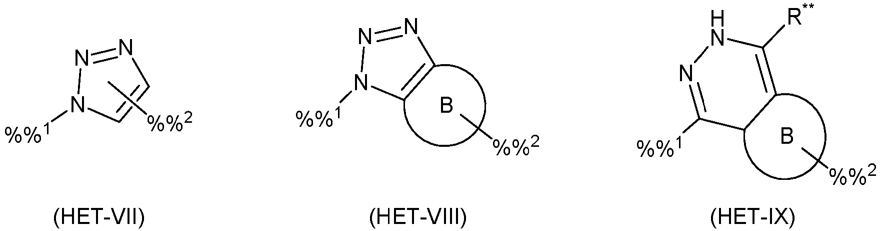 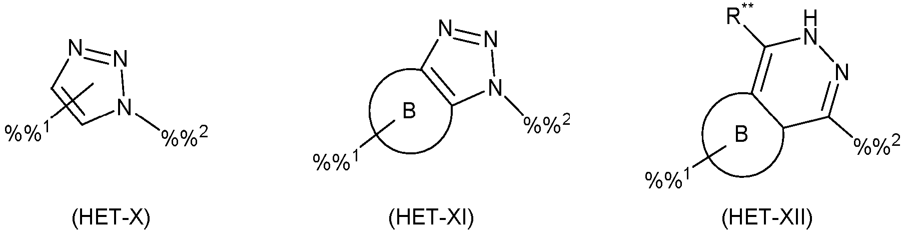
wherein
%%¹ represents the attachment to L⁵ or, when L⁵ is absent, corresponds to P¹,
%%² represents the attachment to L⁶ or, when L⁶ is absent, corresponds to ##¹,
R** is H, an optionally substituted alkyl group or an optionally substituted aryl group, and preferably is a C1-C6 alkyl group,
ring B is an optionally substituted 8-membered carbocycle or an optionally substituted 8-membered heterocycle, and is preferably an optionally substituted dibenzo-fused 8-membered N-heterocycle; and
##¹ represents attachment to the molecular probe.

5. A surface-functionalized optical element comprising
an IR-transparent optical element,
wherein at least a part of the surface of the IR-transparent optical element is bound to a molecular probe by a linker construct,
wherein the linker construct comprises
a surface attachment moiety,
a spacer moiety which comprises a poly(alkylene oxide) and a ligation group, which is covalently bound between the poly(alkylene oxide) of the spacer moiety and the molecular probe,
and wherein the ligation group is an optionally substituted N-heterocyclic group having a structure according to any one of formula (HET-I) to (HET-VI): 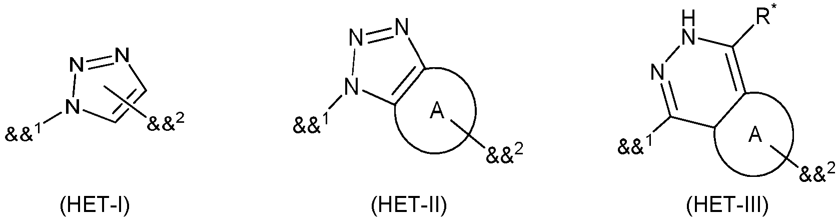 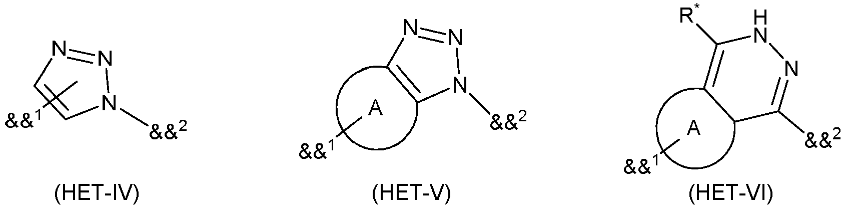
wherein
$$¹ represents attachment to a linking unit of the spacer moiety or to the poly(alkylene oxide),
$$² represents the attachment to a linking unit of the spacer moiety, another moiety of the linker construct or to the molecular probe,
R* is H, an optionally substituted alkyl group or an optionally substituted aryl group, and
ring A is an optionally sustituted 8-membered carbocycle or an optionally sbstituted 8- membered heterocycle.

6. The surface-functionalized optical element according to according to any one of the preceding claims, wherein the IR-transparent optical element is an attenuated total reflection infrared (ATR-IR) waveguide.

7. The surface-functionalized optical element according to any one of the preceding claims, wherein at least a part of the IR-transparent optical element has an oxide surface layer, and wherein at least a part of the oxide surface layer is covalently bound to the linker construct.

8. The surface functionalized optical element according to any one of the preceding claims, wherein the linker construct covalently binds the molecular probe to the surface of the IR- transparent optical element.

9. The surface-functionalized optical element according to any one of the preceding claims, wherein the poly(alkylene oxide) being part of the spacer moiety is a polyethylene glycol, preferably having 2 to 100 glycol repating units, more preferably 2 to 50 glycol repeating units, even more preferably 2 to 25 glycol repeating units, and yet even more preferably 2 to 26 glycol repeating units.

10. The surface-functionalized optical element according to any one of claims 5 to 9, wherein the linker construct has a structure according to formula (A):
//-(S)-(SP)-\\ (A),
wherein
// represents attachment to the surface of the IR-transparent optical element,
(S) represents the surface attachment moiety,
(SP) represents the spacer moiety, and
\\ represents attachment to another moiety of the linker construct or to the molecular probe,
and wherein
the surface attachment moiety (S) has a structure according to formula (S-I):
//-Y¹-L¹-X¹-$$¹ (S-I),
wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is an optional linking unit,
X¹ is a group selected from -C(O)NH-, -NHC(O)-, -NHC(O)HN-, and -NHC(O)O-,
$$¹ represents attachment to the spacer moiety.

11. The surface-functionalized optical element according to any one of claims 5 to 10, wherein the linker construct has a structure according to formula (A):
//-(S)-(SP)-\\ (A),
wherein
// represents attachment to the surface of the IR-transparent optical element,
(S) represents the surface attachment moiety,
(SP) represents the spacer moiety, and
\\ represents attachment to another moiety of the linker construct or to the molecular probe,
and wherein
the spacer moiety (SP) has a structure according to formula (SP-II)
$$²-L²-X²-L³-H¹-L⁴-§§¹ (SP-II),
wherein
$$² represents attachment to the surface attachment moiety (S),
L² to L⁴ is each independently an optional linking unit,
X² is -(OCH₂CH₂)n¹-, wherein n1 is an integer from 2 to 100, preferably from 2 to 50, and more preferably from 2 to 25,
H¹ is the optionally substituted N-heterocyclic group having a structure according to any one of formula (HET-I) to (HET-VI): 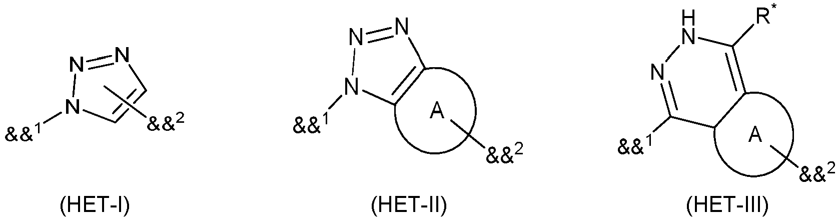 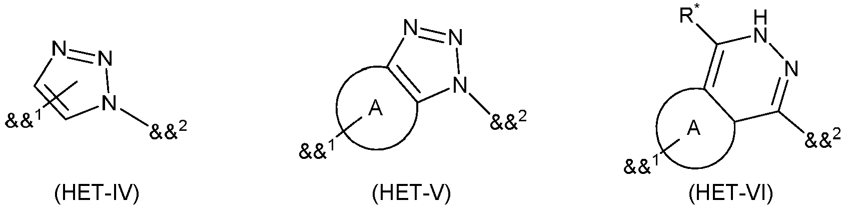
wherein
&&¹ represents attachment to L³ or, if L³ is absent, corresponds to X²,
&&² represents the attachment to L⁴ or, if L⁴ is absent, corresponds to §§¹,
R* is H, an optionally substituted alkyl group or an optionally substituted aryl group, and preferably is a C1-C6 alkyl group,
ring A is an optionally substituted 8-membered carbocycle or an optionally substituted 8-membered heterocycle, and is preferably an optionally substituted dibenzo-fused 8-membered N-heterocycle;
§§¹ represents the attachment to another moiety of the linker construct or to the molecular probe.

12. The surface-functionalized optical element according to any one of claims 5 to 11, wherein the linker construct comprises a peptide moiety, which is covalently bound between the spacer moiety and the molecular probe,
and preferably wherein the linker construct has a structure according to formula (B):
//-(S)-(SP)-(Pep)-\\ (B),
wherein
// represents attachment to the surface of the IR-transparent optical element,
(S) represents the surface attachment moiety,
(SP) represents the spacer moiety,
(PEP) is a peptide moiety,
\\ represents attachment to the molecular probe,
and
wherein the peptide moiety (Pep) has a structure according to Formula (PEP-I):
§§²-P¹-L⁵-H²-L⁶-##¹ (PEP-I),
wherein
§§² represents attachment to the spacer moiety (SP),
P¹ is a peptide compound which is selected from the group consisting of peptides, proteins, protein fragments, each of which is optionally substituted,
L⁵ and L⁶ is each independently an optional linking unit,
H² is an optionally substituted N-heterocyclic group having a structure according to any one of formula (HET-VII) to (HET-XII): 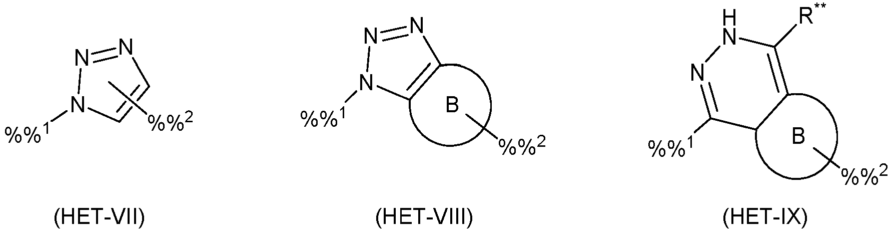 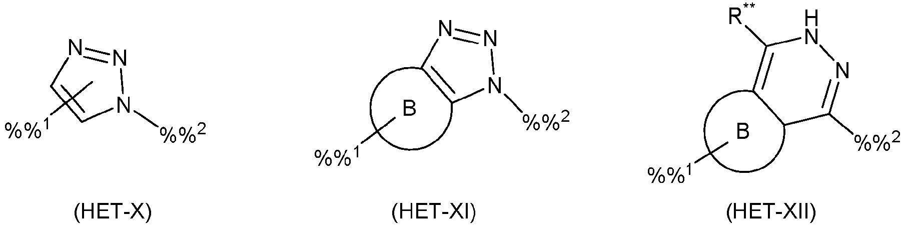
wherein
%%¹ represents the attachment to L⁵ or, when L⁵ is absent, corresponds to P¹,
%%² represents the attachment to L⁶ or, when L⁶ is absent, corresponds to ##¹,
R** is H, an optionally substituted alkyl group or an optionally substituted aryl group, and preferably is a C1-C6 alkyl group,
ring B is an optionally substituted 8-membered carbocycle or an optionally substituted 8-membered heterocycle, and is preferably an optionally substituted dibenzo-fused 8-membered N-heterocycle; and
##¹ represents attachment to the molecular probe.

13. The surface-functionalized optical element according to any one of the preceding claims, wherein the molecular probe is an antigen, an antibody, a fragment of an antibody, an antibody fusion protein or fusion proteins with antibody fragments, an antibody conjugate or conjugates with antibody fragments, a protein complex which optionally contains an antibody fragment or a fusion protein thereof, an anticalin, a nanobody, a small organic molecule, a drug, a nucleic acid, an aptamer, a lipid, a carbohydrate, or a peptide, and/or
wherein the molecular probe is capable of forming a complex with a protein which is associated with a protein misfolding disease.

14. The surface-functionalized optical element according to any one of the preceding claims, further comprising a matrix silane, wherein a part of the surface of the IR-transparent optical element is covalently bound to the matrix silane, and wherein the matrix silane has a structure according to formula (MA-a):
II-(S)-(PAO spacer)-(cap) (MA-a)
wherein
"//" represents attachment to the surface of the IR-transparent optical element,
(S) represents a surface attachment moiety,
(PAO spacer) represents a spacer moiety which comprises a poly(alkylene oxide) group, and
(cap) represents an inert terminal group, and preferably a terminal C1-C6 alkyl group or a terminal C1-C6 alkoxy group.

15. An optical biosensor comprising the surface-functionalized optical element according to any one of claim 1 to 14.

16. A process for preparing a surface-functionalized optical element according to any one of claims 1 to 4, 6 to 9 and 13 to 14, wherein the process comprises the steps of:
(a) providing an IR-transparent optical element,
(b) reacting at least a part of the surface of the IR-transparent optical element with a poly(alkylene oxide)-containing compound comprising a group selected from bioorthogonal-reactive groups to provide an IR-transparent optical element which is at least partially pre-functionalized by a group selected from bioorthogonal-reactive groups,
(c-1) reacting the pre-functionalized optical element obtained in step (b) with a linking unit precursor comprising
(i) a bioorthogonal reactive-group which is suitable for reacting with a bioorthogonal reactive group of the pre-functionalized surface of the optical element, and
(ii) a nucleophile reactive group, optionally selected from aldehydes, carboxylic acids, esters, active esters and maleimide,
(c-3) reacting a peptide compound with the optical element obtained in step (c-1) to obtain an optical element having a surface which is at least partially functionalized by a peptide compound,
(c-4) reacting the peptide compound of the peptide-functionalized optical element obtained in step with a second linking unit precursor,
(c-5) reacting the pre-functionalized surface provided in step (c-4) with a molecular probe to provide the surface-functionalized optical element.

17. A method for detecting a biomarker, comprising the steps of
(i) bringing into contact a surface-functionalized optical element according to any one of claims 1 to 14 with a sample suspected to comprise a target analyte, and optionally wherein the sample is a human body fluid;
(ii) detecting the biomarker based on an interaction between the molecular probe and the target analyte, and optionally wherein the interaction between the molecular probe and the target analyte is detected by infrared spectroscopy, and optionally wherein the biomarker is based on an amide I band position of the target analyte.

18. Use of a surface-functionalized optical element according to any one of claims 1 to 14 or of an optical biosensor according to claim 15 for one or more of:
a companion diagnostic test,
diagnosing a protein misfolding disease, diabetes or a tumor in a patient,
monitoring of therapy of patients having a protein misfolding disease, diabetes or a tumor, and
for screening of drugs for the treatment of a protein misfolding disease, diabetes or a tumor.

## Patentansprüche

1. Ein oberflächenfunktionalisiertes optisches Element, umfassend
ein IR-transparentes optisches Element,
wobei mindestens ein Teil der Oberfläche des IR-transparenten optischen Elements über ein Linker-Konstrukt an eine molekulare Sonde gebunden ist,
wobei das Linker-Konstrukt umfasst
eine Oberflächenbindungsgruppe,
eine Spacer-Gruppe, die ein Poly(alkylenoxid) umfasst, und
eine Peptidgruppe, die kovalent zwischen der Spacer-Gruppe und der molekularen Sonde gebunden ist.

2. Das oberflächenfunktionalisierte optische Element gemäß Anspruch 1, wobei das Linker-Konstrukt eine Struktur gemäß Formel (B) aufweist:
//-(S)-(SP)-(Pep)-\\ (B),
wobei
// die Bindung an die Oberfläche des IR-transparenten optischen Elements darstellt,
(S) die Oberflächenbindungsgruppe darstellt,
(SP) die Spacer-Gruppe darstellt,
(Pep) die Peptidgruppe ist, die eine Peptidverbindung umfasst, welche mindestens zwei Aminosäuren enthält, die durch eine Peptidbindung verbunden sind, und
\\ die Bindung an die Peptidgruppe des Linker-Konstrukts darstellt,
wobei die Oberflächenbindungsgruppe (S) eine Struktur gemäß der Formel (S-I) aufweist:
//-Y¹-L¹-X¹-$$¹ (S-I),
wobei
// die Bindung an die Oberfläche des IR-transparenten optischen Elements darstellt,
Y¹ -O-Si(R¹)₂- ist, wobei R¹ jeweils unabhängig voneinander aus H, OH, gegebenenfalls substituiertem Alkyl, gegebenenfalls substituiertem Alkoxy, -O-// und -O-Si^{x} ausgewählt wird, wobei Si^{x} ein Siliziumatom einer anderen siliziumhaltigen Oberflächenbindungseinheit oder ein Siliziumatom eines optionalen Matrixsilans ist, das an die Oberfläche des IR-transparenten optischen Elements gebunden ist,
L¹ eine optionale Verknüpfungseinheit ist,
X¹ eine Gruppe ist, ausgewählt aus -C(O)NH-, -NHC(O)-, -NHC(O)HN- und -NHC(O)O-,
$$¹ die Bindung an die Spacer-Gruppe darstellt.

3. Das oberflächenfunktionalisierte optische Element gemäß Anspruch 1 oder Anspruch 2, wobei das Linker-Konstrukt eine Struktur gemäß Formel (B) aufweist:
//-(S)-(SP)-(Pep)-\\ (B),
wobei
// steht für die Anbindung an die Oberfläche des IR-transparenten optischen Elements,
(S) steht für die Oberflächenbindungsgruppe,
(SP) steht für die Spacer-Gruppe,
(Pep) die Peptidgruppe ist, die eine Peptidverbindung umfasst, welche mindestens zwei Aminosäuren enthält, die durch eine Peptidbindung verbunden sind, und
\\ die Bindung an die Peptidgruppe des Linker-Konstrukts darstellt,
wobei die Spacer-Gruppe (SP) eine Struktur gemäß der Formel (SP-II) aufweist
$$²-L²-X²-L³-H¹-L⁴-§§¹ (SP-II),
wobei
$$² die Bindung an die Oberflächenbindungseinheit (S) darstellt,
L² bis L⁴ jeweils unabhängig voneinander eine optionale Verknüpfungseinheit sind,
X² ist -(OCH₂CH₂)n¹-, wobei n1 eine ganze Zahl von 2 bis 100, vorzugsweise von 2 bis 50 und noch bevorzugter von 2 bis 25 ist,
H¹ eine gegebenenfalls substituierte N-heterocyclische Gruppe mit einer Struktur gemäß einer der Formeln (HET-I) bis (HET-VI) ist: 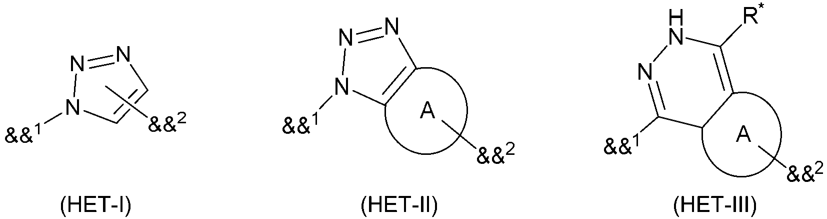 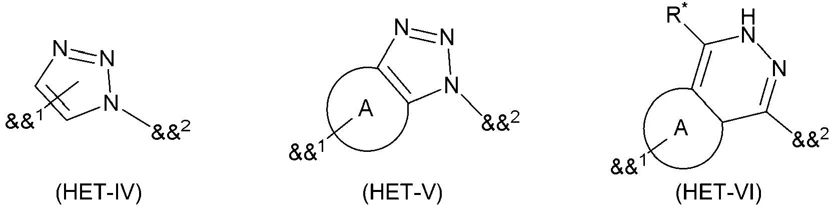
wobei
&&¹ die Bindung an L³ darstellt oder, falls L³ fehlt, X² entspricht,
&&² die Bindung an L⁴ darstellt oder, falls L⁴ fehlt, §§¹ entspricht,
R* H, eine gegebenenfalls substituierte Alkylgruppe oder eine gegebenenfalls substituierte Arylgruppe ist und vorzugsweise eine C1-C6-Alkylgruppe ist,
Ring A ist ein gegebenenfalls substituierter 8-gliedriger Carbocyclus oder ein gegebenenfalls substituierter 8-gliedriger Heterocyclus und ist vorzugsweise ein gegebenenfalls substituierter dibenzokondensierter 8-gliedriger N-Heterocyclus;
§§¹ stellt die Bindung an die Peptidgruppe des Linker-Konstrukts dar.

4. Das oberflächenfunktionalisierte optische Element gemäß einem der Ansprüche 1 bis 3, wobei das Linker-Konstrukt eine Struktur gemäß der Formel (B) aufweist:
//-(S)-(SP)-(Pep)-\\ (B),
wobei
// die Bindung an die Oberfläche des IR-transparenten optischen Elements darstellt,
(S) die Oberflächenbindungsgruppe darstellt,
(SP) die Spacer-Gruppe darstellt,
(Pep) die Peptidgruppe ist,
\\ die Bindung an die molekulare Sonde darstellt,
und wobei die Peptidgruppe (Pep) eine Struktur gemäß Formel (PEP-I) aufweist:
§§²-P¹-L⁵-H²-L⁶-##¹ (PEP-I),
wobei
§§² die Bindung an die Spacer-Gruppe (SP) darstellt,
P¹ eine Peptidverbindung ist, die aus der Gruppe ausgewählt ist, bestehend aus Peptiden, Proteinen und Proteinfragmenten, von denen jedes gegebenenfalls substituiert ist,
L⁵ und L⁶ jeweils unabhängig voneinander eine optionale Verknüpfungseinheit sind,
H² eine gegebenenfalls substituierte N-heterocyclische Gruppe mit einer Struktur gemäß einer der Formeln (HET-VII) bis (HET-XII) ist: 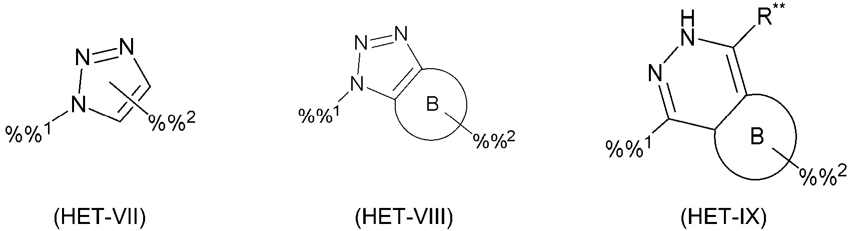 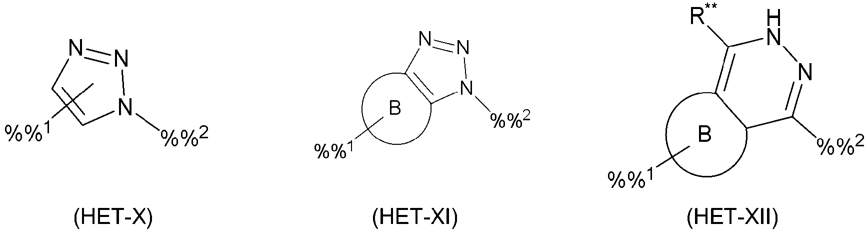
wobei
%%¹ die Bindung an L⁵ darstellt oder, wenn L⁵ fehlt, P¹ entspricht,
%%² die Bindung an L⁶darstellt oder, wenn L⁶ fehlt, ##¹ entspricht,
R** H, eine gegebenenfalls substituierte Alkylgruppe oder eine gegebenenfalls substituierte Arylgruppe ist und vorzugsweise eine C1-C6-Alkylgruppe ist,
Ring B ist ein gegebenenfalls substituierter 8-gliedriger Carbocyclus oder ein gegebenenfalls substituierter 8-gliedriger Heterocyclus und ist vorzugsweise ein gegebenenfalls substituierter dibenzokondensierter 8-gliedriger N-Heterocyclus; und
##¹ die Bindung an die molekulare Sonde darstellt.

5. Ein oberflächenfunktionalisiertes optisches Element, umfassend
ein IR-transparentes optisches Element,
wobei mindestens ein Teil der Oberfläche des IR-transparenten optischen Elements über ein Linker-Konstrukt an eine molekulare Sonde gebunden ist,
wobei das Linker-Konstrukt umfasst
eine Oberflächenbindungsgruppe,
eine Spacer-Gruppe, die ein Poly(alkylenoxid) und eine Ligationsgruppe umfasst, die kovalent zwischen dem Poly(alkylenoxid) der Spacer-Gruppe und der molekularen Sonde gebunden ist,
und wobei die Ligationsgruppe eine gegebenenfalls substituierte N-heterocyclische Gruppe mit einer Struktur gemäß einer der Formeln (HET-I) bis (HET-VI) ist: 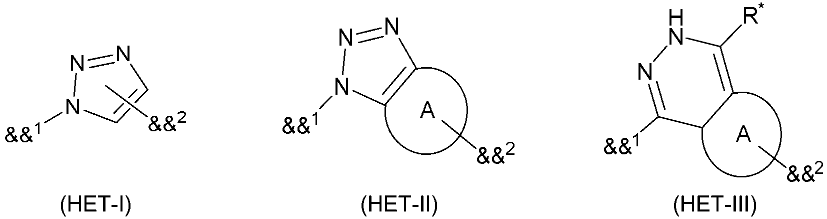 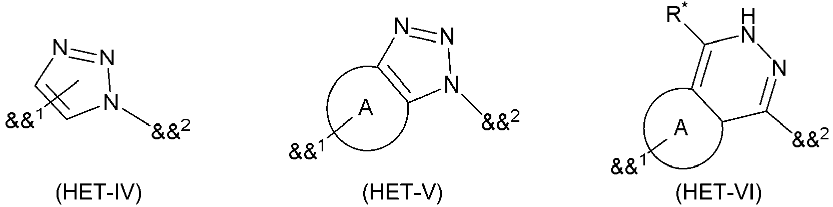
wobei
&&¹ die Bindung an eine Verknüpfungseinheit der Spacer-Einheit oder an das Poly(alkylenoxid) darstellt,
&&² die Bindung an eine Verknüpfungseinheit der Spacer-Einheit, eine andere Einheit des Linker-Konstrukts oder an die molekulare Sonde darstellt,
R* steht für H, eine gegebenenfalls substituierte Alkylgruppe oder eine gegebenenfalls substituierte Arylgruppe, und
Ring A ein gegebenenfalls substituierter 8-gliedriger Carbocyclus oder ein gegebenenfalls substituierter 8-gliedriger Heterocyclus ist.

6. Das oberflächenfunktionalisierte optische Element gemäß einem der vorstehenden Ansprüche, wobei das IR-transparente optische Element ein *attenuated total reflection infrared* (ATR-IR) Wellenleiter ist.

7. Das oberflächenfunktionalisierte optische Element gemäß einem der vorstehenden Ansprüche, wobei zumindest ein Teil des IR-transparenten optischen Elements eine Oxid-Oberflächenschicht aufweist und wobei zumindest ein Teil der Oxid-Oberflächenschicht kovalent an das Linker-Konstrukt gebunden ist.

8. Das oberflächenfunktionalisierte optische Element gemäß einem der vorstehenden Ansprüche, wobei das Linker-Konstrukt die molekulare Sonde kovalent an die Oberfläche des IR-transparenten optischen Elements bindet.

9. Das oberflächenfunktionalisierte optische Element gemäß einem der vorstehenden Ansprüche, wobei das Poly(alkylenoxid), das Teil der Spacer-Einheit ist, ein Polyethylenglykol ist, vorzugsweise mit 2 bis 100 Glykol-Wiederholungseinheiten, noch bevorzugter mit 2 bis 50 Glykol-Wiederholungseinheiten, noch bevorzugter mit 2 bis 25 Glykol-Wiederholungseinheiten und sogar noch bevorzugter mit 2 bis 16 Glykol-Wiederholungseinheiten.

10. Das oberflächenfunktionalisierte optische Element nach einem der Ansprüche 5 bis 9, wobei das Linker-Konstrukt eine Struktur gemäß der Formel (A) aufweist:
//-(S)-(SP)-\\ (A),
wobei
// die Bindung an die Oberfläche des IR-transparenten optischen Elements darstellt,
(S) die Oberflächenbindungsgruppe darstellt,
(SP) die Spacer-Gruppe darstellt und
\\ die Bindung an eine andere Einheit des Linker-Konstrukts oder an die molekulare Sonde darstellt,
und wobei
die Oberflächenbindungsgruppe (S) eine Struktur gemäß der Formel (S-I) aufweist:
//-Y¹-L¹-X¹-$$¹ (S-I),
wobei
// die Bindung an die Oberfläche des IR-transparenten optischen Elements darstellt,
Y¹ -O-Si(R¹)₂- ist, wobei R¹ jeweils unabhängig voneinander aus H, OH, gegebenenfalls substituiertem Alkyl, gegebenenfalls substituiertem Alkoxy, -O-// und -O-Si^{x} ausgewählt wird, wobei Si^{x} ein Siliziumatom einer anderen siliziumhaltigen Oberflächenbindungseinheit oder ein Siliziumatom eines optionalen Matrixsilans ist, das an die Oberfläche des IR-transparenten optischen Elements gebunden ist,
L¹ eine optionale Verknüpfungseinheit ist,
X¹ eine Gruppe ist, ausgewählt aus -C(O)NH-, -NHC(O)-, -NHC(O)HN- und -NHC(O)O-,
$$¹ die Bindung an die Spacer-Gruppe darstellt.

11. Das oberflächenfunktionalisierte optische Element gemäß einem der Ansprüche 5 bis 10, wobei das Linker-Konstrukt eine Struktur gemäß der Formel (A) aufweist:
//-(S)-(SP)-\\ (A),
wobei
// die Bindung an die Oberfläche des IR-transparenten optischen Elements darstellt,
(S) die Oberflächenbindungsgruppe darstellt,
(SP) die Spacer-Gruppe darstellt und
\\ steht für die Bindung an einen anderen Teil des Linker-Konstrukts oder an die molekulare Sonde,
und wobei
der Spacer-Teil (SP) eine Struktur gemäß der Formel (SP-II) aufweist
$$²-L²-X²-L³-H¹-L⁴-§§¹ (SP-II),
wobei
$$² die Bindung an die Oberflächenbindungseinheit (S) darstellt,
L² bis L⁴ jeweils unabhängig voneinander eine optionale Verknüpfungseinheit sind,
X² ist -(OCH₂CH₂)n¹ -, wobei n1 eine ganze Zahl von 2 bis 100, vorzugsweise von 2 bis 50 und noch bevorzugter von 2 bis 25 ist,
H¹ die gegebenenfalls substituierte N-heterocyclische Gruppe mit einer Struktur gemäß einer der Formeln (HET-I) bis (HET-VI) ist: 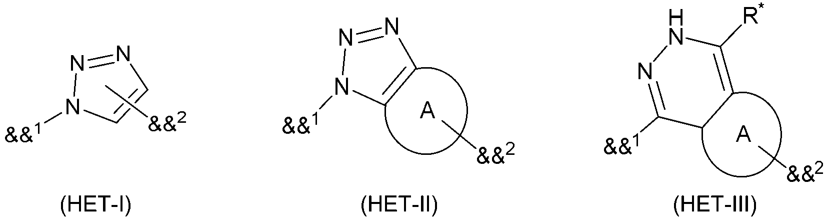 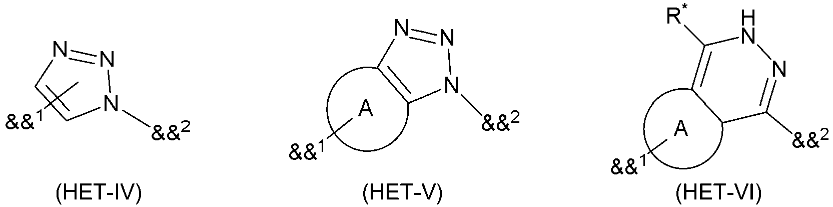
wobei
&&¹ die Anbindung an L³ darstellt oder, falls L³ fehlt, X² entspricht,
&&² die Bindung an L⁴ darstellt oder, falls L⁴ fehlt, §§¹ entspricht,
R* H, eine gegebenenfalls substituierte Alkylgruppe oder eine gegebenenfalls substituierte Arylgruppe ist und vorzugsweise eine C1-C6-Alkylgruppe ist,
Ring A ist ein gegebenenfalls substituierter 8-gliedriger Carbocyclus oder ein gegebenenfalls substituierter 8-gliedriger Heterocyclus und ist vorzugsweise ein gegebenenfalls substituierter dibenzokondensierter 8-gliedriger N-Heterocyclus;
§§¹ stellt die Bindung an eine andere Einheit des Linker-Konstrukts oder an die molekulare Sonde dar.

12. Das oberflächenfunktionalisierte optische Element gemäß einem der Ansprüche 5 bis 11, wobei das Linker-Konstrukt eine Peptidgruppe umfasst, die kovalent zwischen der Spacergruppe und der molekularen Sonde gebunden ist,
und wobei das Linker-Konstrukt vorzugsweise eine Struktur gemäß Formel (B) aufweist:
//-(S)-(SP)-(Pep)-\\ (B),
wobei
// die Bindung an die Oberfläche des IR-transparenten optischen Elements darstellt,
(S) die Oberflächenbindungsgruppe darstellt,
(SP) die Spacer-Gruppe darstellt,
(PEP) eine Peptidgruppe ist,
\\ die Bindung an die molekulare Sonde darstellt,
und
wobei die Peptidgruppe (Pep) eine Struktur gemäß Formel (PEP-I) aufweist:
§§²-P¹-L⁵-H²-L⁶-##¹ (PEP-I),
wobei
§§² die Bindung an die Spacer-Einheit (SP) darstellt,
P¹ eine Peptidverbindung ist, die aus der Gruppe ausgewählt ist, bestehend aus Peptiden, Proteinen und Proteinfragmenten, von denen jedes gegebenenfalls substituiert ist,
L⁵ und L⁶ jeweils unabhängig voneinander eine optionale Verknüpfungseinheit sind,
H² eine gegebenenfalls substituierte N-heterocyclische Gruppe mit einer Struktur gemäß einer der Formeln (HET-VII) bis (HET-XII) ist: 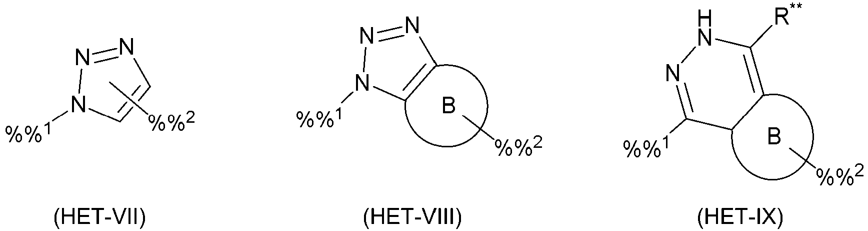 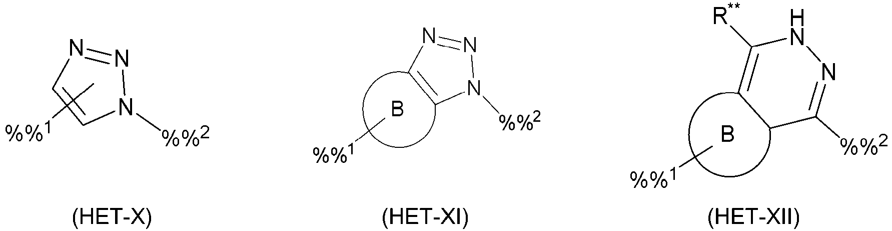
wobei
%%¹ die Bindung an L⁵ darstellt oder, wenn L⁵ fehlt, P¹ entspricht,
%%² die Bindung an L⁶ darstellt oder, wenn L⁶ fehlt, ##¹ entspricht,
R** H, eine gegebenenfalls substituierte Alkylgruppe oder eine gegebenenfalls substituierte Arylgruppe ist und vorzugsweise eine C1-C6-Alkylgruppe ist,
Ring B ist ein gegebenenfalls substituierter 8-gliedriger Carbocyclus oder ein gegebenenfalls substituierter 8-gliedriger Heterocyclus und ist vorzugsweise ein gegebenenfalls substituierter dibenzokondensierter 8-gliedriger N-Heterocyclus; und
##¹ die Bindung an die molekulare Sonde darstellt.

13. Das oberflächenfunktionalisierte optische Element gemäß einem der vorstehenden Ansprüche, wobei die molekulare Sonde ein Antigen, ein Antikörper, ein Fragment eines Antikörpers, ein Antikörper-Fusionsprotein oder Fusionsproteine mit Antikörperfragmenten, ein Antikörperkonjugat oder Konjugate mit Antikörperfragmenten, ein Proteinkomplex, der gegebenenfalls ein Antikörperfragment oder ein Fusionsprotein davon enthält, ein Anticalin, ein Nanobody, ein kleines organisches Molekül, ein Arzneimittel, eine Nukleinsäure, ein Aptamer, ein Lipid, ein Kohlenhydrat oder ein Peptid ist, und/oder
wobei die molekulare Sonde in der Lage ist, einen Komplex mit einem Protein zu bilden, das mit einer durch Fehlfaltung von Proteinen verursachten Erkrankung assoziiert ist.

14. Das oberflächenfunktionalisierte optische Element gemäß einem der vorstehenden Ansprüche, das ferner ein Matrixsilan umfasst, wobei ein Teil der Oberfläche des IR-transparenten optischen Elements kovalent an das Matrixsilan gebunden ist und wobei das Matrixsilan eine Struktur gemäß der Formel (MA-a) aufweist:
//-(S)-(PAO-Spacer)-(Cap) (MA-a)
wobei
"//" die Bindung an die Oberfläche des IR-transparenten optischen Elements darstellt,
(S) eine Oberflächenbindungsgruppe darstellt,
(PAO-Spacer) eine Spacer-Gruppe darstellt, die eine Poly(alkylenoxid)-Gruppe umfasst, und
(Cap) eine inerte Endgruppe darstellt, vorzugsweise eine endständige C1-C6-Alkylgruppe oder eine endständige C1-C6-Alkoxygruppe.

15. Ein optischer Biosensor, der das oberflächenfunktionalisierte optische Element gemäß einem der Ansprüche 1 bis 14 umfasst.

16. Ein Verfahren zur Herstellung eines oberflächenfunktionalisierten optischen Elements gemäß einem der Ansprüche 1 bis 4, 6 bis 9 und 13 bis 14, wobei das Verfahren die folgenden Schritte umfasst:
(a) Bereitstellen eines IR-transparenten optischen Elements,
(b) Reagieren mindestens eines Teils der Oberfläche des IR-transparenten optischen Elements mit einer Poly(alkylenoxid)-haltigen Verbindung, die eine Gruppe umfasst, die aus bioorthogonal reaktiven Gruppen ausgewählt ist, um ein IR-transparentes optisches Element bereitzustellen, das mindestens teilweise durch eine Gruppe vorfunktionalisiert ist, die aus bioorthogonal reaktiven Gruppen ausgewählt ist,
(c-1) Umsetzen des in Schritt (b) erhaltenen vorfunktionalisierten optischen Elements mit einem Vorläufer einer Verknüpfungseinheit, der Folgendes umfasst
(i) eine bioorthogonal-reaktive Gruppe, die zur Reaktion mit einer bioorthogonalen reaktiven Gruppe der vorfunktionalisierten Oberfläche des optischen Elements geeignet ist, und
(ii) eine Nukleophil-reaktive Gruppe, die gegebenenfalls aus Aldehyden, Carbonsäuren, Estern, aktiven Estern und Maleimid ausgewählt ist,
(c-3) Reagieren einer Peptidverbindung mit dem in Schritt (c-1) erhaltenen optischen Element, um ein optisches Element zu erhalten, dessen Oberfläche zumindest teilweise durch eine Peptidverbindung funktionalisiert ist,
(c-4) Umsetzen der Peptidverbindung des in Schritt erhaltenen peptidfunktionalisierten optischen Elements mit einem Vorläufer einer zweiten Verknüpfungseinheit,
(c-5) Reagieren der in Schritt (c-4) bereitgestellten vorfunktionalisierten Oberfläche mit einer molekularen Sonde, um das oberflächenfunktionalisierte optische Element bereitzustellen.

17. Ein Verfahren zum Nachweis eines Biomarkers, umfassend die Schritte
(i) Inkontaktbringen eines oberflächenfunktionalisierten optischen Elements gemäß einem der Ansprüche 1 bis 14 mit einer Probe, von der vermutet wird, dass sie einen Zielanalyten enthält, wobei die Probe gegebenenfalls eine menschliche Körperflüssigkeit ist;
(ii) Nachweisen des Biomarkers auf der Grundlage einer Wechselwirkung zwischen der molekularen Sonde und dem Zielanalyten, wobei die Wechselwirkung zwischen der molekularen Sonde und dem Zielanalyten optional mittels Infrarotspektroskopie nachgewiesen wird und der Biomarker optional auf der Position einer Amid-I-Bande des Zielanalyten basiert.

18. Eine Verwendung eines oberflächenfunktionalisierten optischen Elements gemäß einem der Ansprüche 1 bis 14 oder eines optischen Biosensors gemäß Anspruch 15 für eines oder mehrere der folgenden Zwecke:
einen Begleitdiagnostiktest,
zur Diagnose einer durch Fehlfaltung von Proteinen verursachten Erkrankung, von Diabetes oder eines Tumors bei einem Patienten,
Überwachung der Therapie von Patienten mit einer durch Fehlfaltung von Proteinen verursachten Erkrankung, mit Diabetes oder einem Tumor sowie
zum Screening von Arzneimitteln zur Behandlung einer durch Fehlfaltung von Proteinen verursachten Erkrankung, von Diabetes oder eines Tumors.

## Revendications

1. Élément optique fonctionnalisé en surface comprenant
un élément optique transparent dans l'IR,
sachant qu'au moins une partie de la surface de l'élément optique transparent dans l'IR est liée à une sonde moléculaire par un bras de liaison,
sachant que le bras de liaison comprend
un groupement de fixation de surface,
un groupement espaceur comprenant un poly(oxyde d'alkylène), et
un groupement peptidique, lequel est lié de manière covalente entre le groupement espaceur et la sonde moléculaire.

2. L'élément optique fonctionnalisé en surface selon la revendication 1, sachant que le bras de liaison présente une structure selon la formule (B) :
//-(S)-(SP)-(Pep)-\\ (B),
sachant que
// représente la fixation à la surface de l'élément optique transparent dans l'IR,
(S) représente le groupement de fixation de surface,
(SP) représente le groupement espaceur,
(Pep) est le groupement peptidique comprenant un composé peptidique qui comprend au moins deux acides aminés qui sont liés par une liaison peptidique, et
\\ représente la fixation au groupement peptidique du bras de liaison,
sachant que le groupement de fixation de surface (S) présente une structure selon la formule (S-I) :
//-Y¹-L¹-X¹-$$¹ (S-I),
sachant que
// représente la fixation à la surface de l'élément optique transparent dans l'IR,
Y¹ est -O-Si(R¹)₂-, sachant que R¹ est sélectionné chacun indépendamment parmi H, OH, un alkyle facultativement substitué, un alcoxy facultativement substitué, -O-//, et -O-Si^{x}, sachant que Si^{x} est un atome de silicium d'un autre groupement de fixation de surface contenant du silicium ou un atome de silicium d'un silane de matrice facultatif qui est fixé à la surface de l'élément optique transparent dans l'IR,
L¹ est une unité de liaison facultative,
X¹ est un groupe sélectionné parmi -C(O)NH-, -NHC(O)-, - NHC(O)HN-, et -NHC(O)O-,
$$¹ représente la fixation au groupement espaceur.

3. L'élément optique fonctionnalisé en surface selon la revendication 1 ou la revendication 2, sachant que le bras de liaison présente une structure selon la formule (B) :
//-(S)-(SP)-(Pep)-\\ (B),
sachant que
// représente la fixation à la surface de l'élément optique transparent dans l'IR,
(S) représente le groupement de fixation de surface,
(SP) représente le groupement espaceur,
(Pep) est le groupement peptidique comprenant un composé peptidique qui comprend au moins deux acides aminés qui sont liés par une liaison peptidique, et
\\ représente la fixation au groupement peptidique du bras de liaison,
sachant que le groupement espaceur (SP) présente une structure selon la formule (SP-II)
$$²-L²-X²-L³-H¹-L⁴-§§¹ (SP-II),
sachant que
$$² représente la fixation au groupement de fixation de surface (S),
L² à L⁴ sont chacun indépendamment une unité de liaison facultative,
X² est -(OCH₂CH₂)n¹-, sachant que n¹ est un entier de 2 à 100, de préférence de 2 à 50, et de manière plus préférentielle de 2 à 25,
H¹ est un groupe N-hétérocyclique facultativement substitué présentant une structure selon l'une quelconque des formules (HET-I) à (HET-VI) : 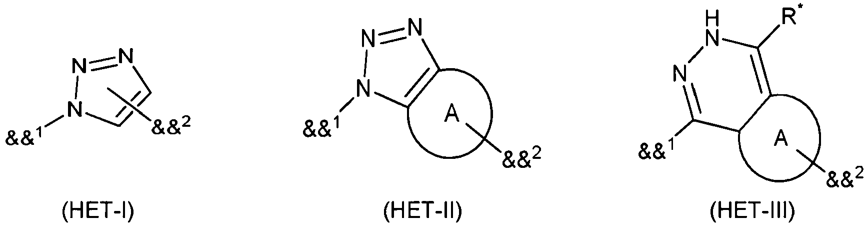 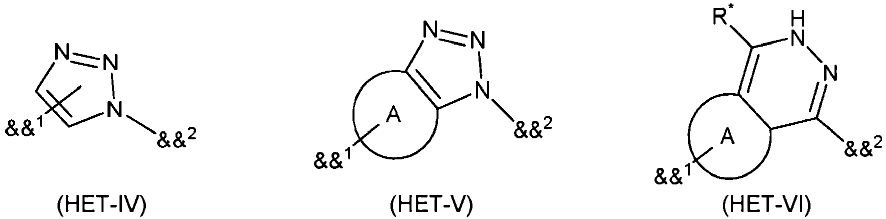
sachant que
&&¹ représente la fixation à L³ ou, si L³ est absent, correspond à X²,
&&² représente la fixation à L⁴ ou, si L⁴ est absent, correspond à §§¹,
R* est H, un groupe alkyle facultativement substitué ou un groupe aryle facultativement substitué, et est de préférence un groupe alkyle en C1-C6,
l'anneau A est un carbocycle à 8 chaînons facultativement substitué ou un hétérocycle à 8 chaînons facultativement substitué, et est de préférence un N-hétérocycle à 8 chaînons dibenzofusionné facultativement substitué ;
§§¹ représente la fixation au groupement peptidique du bras de liaison.

4. L'élément optique fonctionnalisé en surface selon l'une quelconque des revendications 1 à 3, sachant que le bras de liaison présente une structure selon la formule (B) :
//-(S)-(SP)-(Pep)-\\ (B),
sachant que
// représente la fixation à la surface de l'élément optique transparent dans l'IR,
(S) représente le groupement de fixation de surface,
(SP) représente le groupement espaceur,
(Pep) est le groupement peptidique,
\\ représente la fixation à la sonde moléculaire,
et sachant que le groupement peptidique (Pep) présente une structure selon la formule (PEP-I) :
§§²-P¹-L⁵-H²-L⁶-##¹ (PEP-I),
sachant que
§§² représente la fixation au groupement espaceur (SP),
P¹ est un composé peptidique qui est sélectionné dans le groupe constitué par des peptides, des protéines, des fragments de protéine, dont chacun est facultativement substitué,
L⁵ et L⁶ sont chacun indépendamment une unité de liaison facultative,
H² est un groupe N-hétérocyclique facultativement substitué présentant une structure selon l'une quelconque des formules (HET-VII) à (HET-XII) : 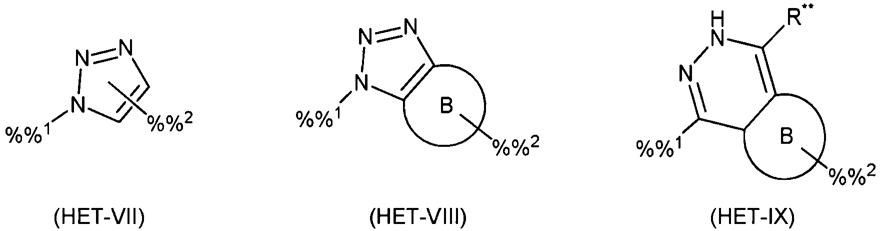 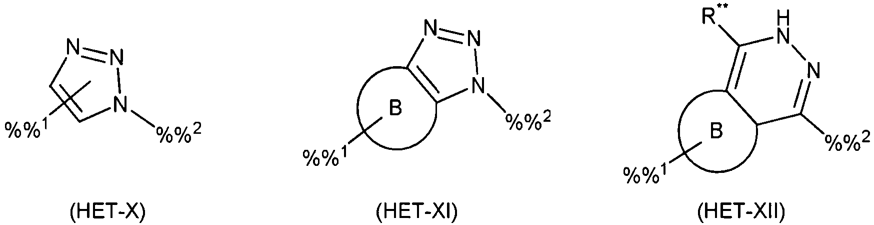
sachant que
%%¹ représente la fixation à L⁵ ou, lorsque L⁵ est absent, correspond à P¹,
%%² représente la fixation à L⁶ ou, lorsque L⁶ est absent, correspond à ##¹,
R** est H, un groupe alkyle facultativement substitué ou un groupe aryle facultativement substitué, et est de préférence un groupe alkyle en C1-C6,
l'anneau B est un carbocycle à 8 chaînons facultativement substitué ou un hétérocycle à 8 chaînons facultativement substitué, et est de préférence un N-hétérocycle à 8 chaînons dibenzofusionné facultativement substitué ; et
##¹ représente la fixation à la sonde moléculaire.

5. Élément optique fonctionnalisé en surface comprenant
un élément optique transparent dans l'IR,
sachant qu'au moins une partie de la surface de l'élément optique transparent dans l'IR est liée à une sonde moléculaire par un bras de liaison,
sachant que le bras de liaison comprend
un groupement de fixation de surface,
un groupement espaceur qui comprend un poly(oxyde d'alkylène) et un groupe de ligature, qui est lié de manière covalente entre le poly(oxyde d'alkylène) du groupement espaceur et la sonde moléculaire,
et sachant que le groupe de ligature est un groupe N-hétérocyclique facultativement substitué présentant une structure selon l'une quelconque des formules (HET-I) à (HET-VI) : 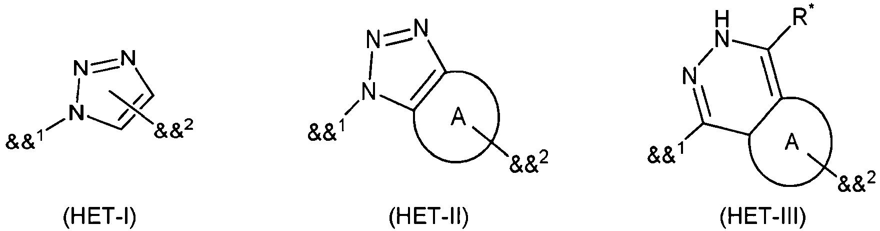 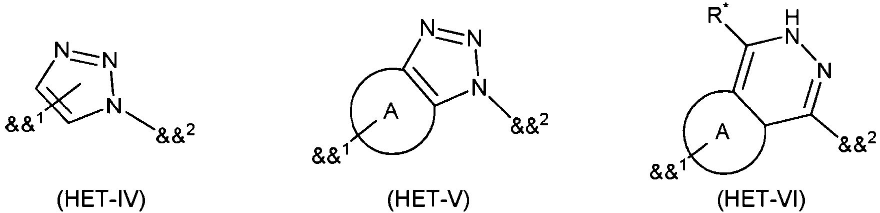
sachant que
&&¹ représente la fixation à une unité de liaison du groupement espaceur ou au poly(oxyde d'alkylène),
&&² représente la fixation à une unité de liaison du groupement espaceur, un autre groupement du bras de liaison ou à la sonde moléculaire,
R* est H, un groupe alkyle facultativement substitué ou un groupe aryle facultativement substitué, et
l'anneau A est un carbocycle à 8 chaînons facultativement substitué ou un hétérocycle à 8 chaînons facultativement substitué.

6. L'élément optique fonctionnalisé en surface selon l'une quelconque des revendications précédentes, sachant que l'élément optique transparent dans l'IR est un guide d'onde infrarouge à réflexion totale atténuée (ATR-IR).

7. L'élément optique fonctionnalisé en surface selon l'une quelconque des revendications précédentes, sachant qu'au moins une partie de l'élément optique transparent dans l'IR présente une couche d'oxyde de surface, et sachant qu'au moins une partie de la couche d'oxyde de surface est liée de manière covalente au bras de liaison.

8. L'élément optique fonctionnalisé en surface selon l'une quelconque des revendications précédentes, sachant que le bras de liaison lie de manière covalente la sonde moléculaire à la surface de l'élément optique transparent dans l'IR.

9. L'élément optique fonctionnalisé en surface selon l'une quelconque des revendications précédentes, sachant que le poly(oxyde d'alkylène) qui fait partie du groupement espaceur est un glycol de polyéthylène, de préférence ayant 2 à 100 unités de répétition de glycol, de manière plus préférentielle 2 à 50 unités de répétition de glycol, de manière encore plus préférentielle 2 à 25 unités de répétition de glycol, et de manière tout particulièrement préférentielle 2 à 16 unités de répétition de glycol.

10. L'élément optique fonctionnalisé en surface selon l'une quelconque des revendications 5 à 9, sachant que le bras de liaison présente une structure selon la formule (A) :
II-(S)-(SP)\\ (A),
sachant que
// représente la fixation à la surface de l'élément optique transparent dans l'IR,
(S) représente le groupement de fixation de surface,
(SP) représente le groupement espaceur, et
\\ représente la fixation à un autre groupement du bras de liaison ou à la sonde moléculaire,
et sachant que
le groupement de fixation de surface (S) présente une structure selon la formule (S-I) :
//-Y¹-L¹-X¹-$$¹ (S-I),
sachant que
// représente la fixation à la surface de l'élément optique transparent dans l'IR,
Y¹ est -O-Si(R¹)₂-, sachant que R¹ est sélectionné chacun indépendamment parmi H, OH, un alkyle facultativement substitué, un alcoxy facultativement substitué, -O-//, et -O-Si^{x}, sachant que Si^{x} est un atome de silicium d'un autre groupement de fixation de surface contenant du silicium ou un atome de silicium d'un silane de matrice facultatif qui est fixé à la surface de l'élément optique transparent dans l'IR,
L¹ est une unité de liaison facultative,
X¹ est un groupe sélectionné parmi -C(O)NH-, -NHC(O)-, - NHC(O)HN-, et -NHC(O)O-,
$$¹ représente la fixation au groupement espaceur.

11. L'élément optique fonctionnalisé en surface selon l'une quelconque des revendications 5 à 10, sachant que le bras de liaison présente une structure selon la formule (A) :
//-(S)-(SP)\\ (A),
sachant que
// représente la fixation à la surface de l'élément optique transparent dans l'IR,
(S) représente le groupement de fixation de surface,
(SP) représente le groupement espaceur, et
\\ représente la fixation à un autre groupement du bras de liaison ou à la sonde moléculaire,
et sachant que
le groupement espaceur (SP) présente une structure selon la formule (SP-II) :
$$²-L²-X²-L³-H¹-L⁴-§§¹ (SP-II),
sachant que
$$² représente la fixation au groupement de fixation de surface (S),
L² à L⁴ sont chacun indépendamment une unité de liaison facultative,
X² est -(OCH₂CH₂)n¹-, sachant que n¹ est un entier de 2 à 100, de préférence de 2 à 50, et de manière plus préférentielle de 2 à 25,
H¹ est le groupe N-hétérocyclique facultativement substitué présentant une structure selon l'une quelconque des formules (HET-I) à (HET-VI) : 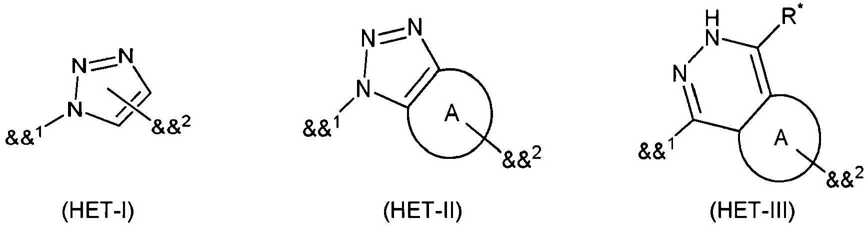 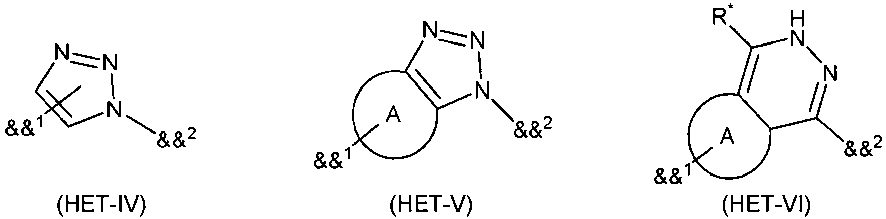
sachant que
&&¹ représente la fixation à L³ ou, si L³ est absent, correspond à X²,
&&² représente la fixation à L⁴ ou, si L⁴ est absent, correspond à §§¹,
R* est H, un groupe alkyle facultativement substitué ou un groupe aryle facultativement substitué, et est de préférence un groupe alkyle en C1-C6,
l'anneau A est un carbocycle à 8 chaînons facultativement substitué ou un hétérocycle à 8 chaînons facultativement substitué, et est de préférence un N-hétérocycle à 8 chaînons dibenzofusionné facultativement substitué ;
§§¹ représente la fixation à un autre groupement du bras de liaison ou à la sonde moléculaire.

12. L'élément optique fonctionnalisé en surface selon l'une quelconque des revendications 5 à 11, sachant que le bras de liaison comprend un groupement peptidique, lequel est lié de manière covalente entre le groupement espaceur et la sonde moléculaire,
et de préférence sachant que le bras de liaison présente une structure selon la formule (B) :
//-(S)-(SP)-(Pep)-\\ (B),
sachant que
// représente la fixation à la surface de l'élément optique transparent dans l'IR,
(S) représente le groupement de fixation de surface,
(SP) représente le groupement espaceur,
(PEP) est un groupement peptidique,
\\ représente la fixation à la sonde moléculaire, et
sachant que le groupement peptidique (Pep) présente une structure selon la formule (PEP-I) :
§§²-P¹-L⁵-H²-L⁶-##¹ (PEP-I),
sachant que
§§² représente la fixation au groupement espaceur (SP),
P¹ est un composé peptidique qui est sélectionné dans le groupe constitué par des peptides, des protéines, des fragments de protéine, dont chacun est facultativement substitué,
L⁵ et L⁶ sont chacun indépendamment une unité de liaison facultative,
H² est un groupe N-hétérocyclique facultativement substitué présentant une structure selon l'une quelconque des formules (HET-VII) à (HET-XII) : 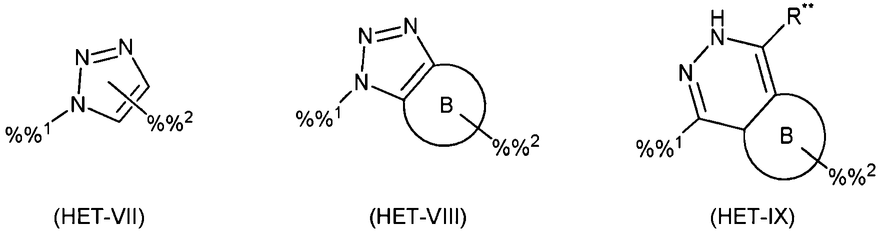 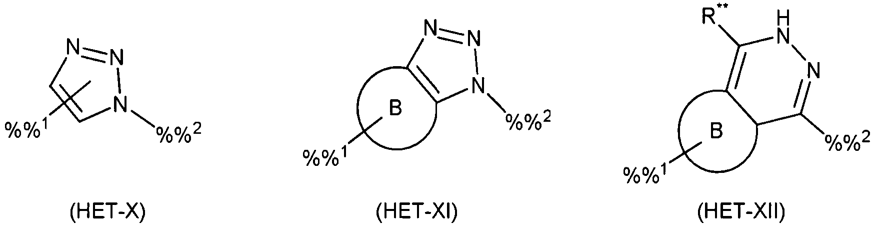
sachant que
%%¹ représente la fixation à L⁵ ou, lorsque L⁵ est absent, correspond à P¹,
%%² représente la fixation à L⁶ ou, lorsque L⁶ est absent, correspond à ##¹,
R** est H, un groupe alkyle facultativement substitué ou un groupe aryle facultativement substitué, et est de préférence un groupe alkyle en C1-C6,
l'anneau B est un carbocycle à 8 chaînons facultativement substitué ou un hétérocycle à 8 chaînons facultativement substitué, et est de préférence un N-hétérocycle à 8 chaînons dibenzofusionné facultativement substitué ; et
##¹ représente la fixation à la sonde moléculaire.

13. L'élément optique fonctionnalisé en surface selon l'une quelconque des revendications précédentes, sachant que la sonde moléculaire est un antigène, un anticorps, un fragment d'un anticorps, une protéine de fusion d'anticorps ou des protéines de fusion avec des fragments d'anticorps, un conjugué d'anticorps ou des conjugués avec des fragments d'anticorps, un complexe protéique qui contient facultativement un fragment d'anticorps ou une protéine de fusion de celui-ci, un anticaline, un nanocorps, une petite molécule organique, un médicament, un acide nucléique, un aptamère, un lipide, un carbohydrate, ou un peptide, et/ou
sachant que la sonde moléculaire est capable de former un complexe avec une protéine qui est associée à une maladie de mauvais repliement des protéines.

14. L'élément optique fonctionnalisé en surface selon l'une quelconque des revendications précédentes, comprenant en outre un silane de matrice, sachant qu'une partie de la surface de l'élément optique transparent dans l'IR est liée de manière covalente au silane de matrice, et sachant que le silane de matrice présente une structure selon la formule (MA-a) :
//-(S)-(PAO spacer) - (cap) (MA-a)
sachant que
« // » représente la fixation à la surface de l'élément optique transparent dans l'IR,
(S) représente un groupement de fixation de surface,
(PAO spacer) représente un groupement espaceur qui comprend un groupe poly(oxyde d'alkylène), et
(cap) représente un groupe terminal inerte, et de préférence un groupe alkyle terminal en C1-C6 ou un groupe alcoxy terminal en C1-C6.

15. Biocapteur optique comprenant l'élément optique fonctionnalisé en surface selon l'une quelconque des revendications 1 à 14.

16. Procédé de préparation d'un élément optique fonctionnalisé en surface selon l'une quelconque des revendications 1 à 4, 6 à 9 et 13 à 14, sachant que le procédé comprend les étapes de :
(a) fourniture d'un élément optique transparent dans l'IR,
(b) réaction d'au moins une partie de la surface de l'élément optique transparent dans l'IR avec un composé contenant un poly(oxyde d'alkylène) comprenant un groupe sélectionné parmi des groupes bioorthogonaux réactifs pour fournir un élément optique transparent dans l'IR qui est au moins en partie préfonctionnalisé par un groupe sélectionné parmi des groupes bioorthogonaux réactifs,
(c-1) réaction de l'élément optique préfonctionnalisé obtenu à l'étape (b) avec un précurseur d'unité de liaison comprenant
(i) un groupe bioorthogonal réactif qui est apte à réagir avec un groupe bioorthogonal réactif de la surface préfonctionnalisée de l'élément optique, et
(ii) un groupe réactif nucléophile, facultativement sélectionné parmi des aldéhydes, des acides carboxyliques, des esters, des esters actifs et la malémide,
(c-3) réaction d'un composé peptidique avec l'élément optique obtenu à l'étape (c-1) pour obtenir un élément optique ayant une surface qui est au moins en partie fonctionnalisée par un composé peptidique,
(c-4) réaction du composé peptidique de l'élément optique fonctionnalisé par un peptide obtenu à l'étape avec un deuxième précurseur d'unité de liaison,
(c-5) réaction de la surface préfonctionnalisée fournie à l'étape (c-4) avec une sonde moléculaire pour fournir l'élément optique fonctionnalisé en surface.

17. Procédé de détection d'un biomarqueur, comprenant les étapes de
(i) mise en contact d'un élément optique fonctionnalisé en surface selon l'une quelconque des revendications 1 à 14 avec un échantillon susceptible de contenir un analyte cible, et facultativement sachant que l'échantillon est un fluide corporel humain ;
(ii) détection du biomarqueur sur la base d'une interaction entre la sonde moléculaire et l'analyte cible, et facultativement sachant que l'interaction entre la sonde moléculaire et l'analyte cible est détectée par spectroscopie infrarouge, et facultativement sachant que le biomarqueur est basé sur une position de bande I d'amide de l'analyte cible.

18. Utilisation d'un élément optique fonctionnalisé en surface selon l'une quelconque des revendications 1 à 14 ou d'un biocapteur optique selon la revendication 15 pour un ou plusieurs de :
un test de diagnostic compagnon,
le diagnostic d'une maladie de mauvais repliement des protéines, d'un diabète ou d'une tumeur chez un patient,
le suivi thérapeutique de patients atteints d'une maladie de mauvais repliement des protéines, d'un diabète ou d'une tumeur, et
le criblage de médicaments pour le traitement d'une maladie de mauvais repliement des protéines, d'un diabète ou d'une tumeur.
